# EUROPEAN PATENT APPLICATION

(11) **EP 3 058 825 A1**
(43) Date of publication of application: **24.08.2016**
(21) Application number: 15382067.5
(22) Date of filing: 19.02.2015
(51) Int. Cl.: A01N 63/00, C12R 1/41

(54) **Bacteria with nematicidal activity**

(71) Applicant: Futureco Bioscience, S.A., 08799 Olèrdola - Barcelona (ES)
(72) Inventor: Lara Sánchez, Jose Manuel, 08799 Olèrdola - Barcelona (ES); Fernández Castillo, Carolina, 08799 Olèrdola - Barcelona (ES)
(74) Representative: ABG Patentes, S.L.

(57) **Abstract**

The invention relates to strains of a microorganism from the species *Ensifer adhaerens* having nematicidal activity against Meloidogyne javanica and to methods for obtaining a biomass of said microorganisms. The invention also provides methods for biologically controlling nematodes and for treating and preventing plant infection caused by nematodes based on the use of a microorganism from the species *Ensifer adhaerens* or of phytosanitary products obtained therefrom.

SEQUENCE LISTING

<110>  FUTURECO BIOSCIENCE, S.A.

<120>  BACTERIA WITH NEMATICIDAL ACTIVITY

<130>  P11575EP00

<160>  9

<170>  PatentIn version 3.5

<210>  1
<211>  18
<212>  DNA
<213>  Artificial Sequence

<220>
<223>  Forward primer 8-f

<400>  1
agtttgatcc tggctcag                                                    18


<210>  2
<211>  21
<212>  DNA
<213>  Artificial Sequence

<220>
<223>  Reverse primer 1492-r

<400>  2
acggttacct tgttacgact t                                                21


<210>  3
<211>  1054
<212>  DNA
<213>  Ensifer adhaerens strain B742

<400>  3
cgagcgcccc gcaaggggag cggcagacgg gtgagtaacg cgtgggaatc taccctttc      60

tacggaataa cgcagggaaa cttgtgctaa taccgtatga gcccttcggg ggaaagattt     120

atcgggaaag gatgagcccg cgttggatta gctagttggt ggggtaaagg cctaccaagg     180

cgacgatcca tagctggtct gagaggatga tcagccacat tgggactgag acacggccca     240

aactcctacg ggaggcagca gtggggaata ttggacaatg ggcgcaagcc tgatccagcc     300

atgccgcgtg agtgatgaag gccctagggt tgtaaagctc tttcaccggt gaagataatg     360

acggtaaccg gagaagaagc cccggctaac ttcgtgccag cagccgcggt aatacgaagg     420

gggctagcgt tgttcggaat tactgggcgt aaagcgcacg taggcggaca tttaagtcag     480

gggtgaaatc ccggggctca accccggaac tgcctttgat actgggtgtc tagagtatgg     540

aagaggtgag tggaattccg agtgtagagg tgaaattcgt agatattcgg aggaacacca     600

gtggcgaagg cggctcactg gtccattact gacgctgagg tgcgaaagcg tggggagcaa     660

acaggattag ataccctggt agtccacgcc gtaaacgatg aatgttagcc gtcgggcagt 720

ttactgttcg gtggcgcagc taacgcatta aacattccgc ctggggagta cggtcgcaag 780

attaaaactc aaaggaattg acgggggccc gcacaagcgg tggagcatgt ggtttaattc 840

gaagcaacgc gcagaacctt accagccctt gacatcccga tcgcggatta cggagacgtt 900

ttccttcagt tcggctggat cggagacagg tgctgcatgg ctgtcgtcag ctcgtgtcgt 960

gagatgttgg gttaagtccc gcaacgagcg caaccctcgc ccttagttgc cagcatttag 1020

ttgggcactc taaggggact gccggtgata agcc 1054

<210> 4
<211> 986
<212> DNA
<213> Ensifer adhaerens strain B758

<400> 4
tgcagtcgag cgccccgcaa ggggagcggc agacgggtga gtaacgcgtg ggaatctacc 60

cttttctacg gaataacgca gggaaacttg tgctaatacc gtataagccc ttcgggggaa 120

agatttatcg ggaaaggatg agcccgcgtt ggattagcta gttggtgggg taaaggccta 180

ccaaggcgac gatccatagc tggtctgaga ggatgatcag ccacattggg actgagacac 240

ggcccaaact cctacgggag gcagcagtgg ggaatattgg acaatgggcg caagcctgat 300

ccagccatgc cgcgtgagtg atgaaggccc tagggttgta aagctctttc accggtgaag 360

ataatgacgg taaccggaga agaagccccg gctaacttcg tgccagcagc cgcggtaata 420

cgaagggggc tagcgttgtt cggaattact gggcgtaaag cgcacgtagg cggacattta 480

agtcaggggt gaaatcccag agctcaactc tggaactgcc tttgatactg ggtgtctaga 540

gtatggaaga ggtgagtgga attccgagtg tagaggtgaa attcgtagat attcggagga 600

acaccagtgg cgaaggcggc tcactggtcc attactgacg ctgaggtgcg aaagcgtggg 660

gagcaaacag gattagatac cctggtagtc cacgccgtaa acgatgaatg ttagccgtcg 720

ggcagtttac tgttcggtgg cgcagctaac gcattaaaca ttccgcctgg ggagtacggt 780

cgcaagatta aaactcaaag gaattgacgg gggcccgcac aagcggtgga gcatgtggtt 840

taattcgaag caacgcgcag aaccttacca gcccttgaca tcccgatcgc ggattacgga 900

gacgttttcc ttcagttcgg ctggatcgga gacaggtgct gcatggctgt cgtcagctcg 960

tgtcgtgaga tgttgggtta agtccc 986

<210> 5
<211> 1048
<212> DNA
<213> Ensifer adhaerens strain B760

<400> 5

agtcgagcgc cccgcaaggg gagcggcaga cgggtgagta acgcgtggga atctaccctt 60

ttctacggaa taacgcaggg aaacttgtgc taataccgta tacgcccttc gggggaaaga 120

tttatcggga aaggatgagc ccgcgttgga ttagctagtt ggtggggtaa aggcctacca 180

aggcgacgat ccatagctgg tctgagagga tgatcagcca cattgggact gagacacggc 240

ccaaactcct acgggaggca gcagtgggga atattggaca atgggcgcaa gcctgatcca 300

gccatgccgc gtgagtgatg aaggccctag ggttgtaaag ctctttcacc ggtgaagata 360

atgacggtaa ccggagaaga agccccggct aacttcgtgc cagcagccgc ggtaatacga 420

agggggctag cgttgttcgg aattactggg cgtaaagcgc acgtaggcgg acatttaagt 480

caggggtgaa atcccggggc tcaaccccgg aactgccttt gatactgggt gtctagagta 540

tggaagaggt gagtggaatt ccgagtgtag aggtgaaatt cgtagatatt cggaggaaca 600

ccagtggcga aggcggctca ctggtccatt actgacgctg aggtgcgaaa gcgtggggag 660

caaacaggat tagataccct ggtagtccac gccgtaaacg atgaatgtta gccgtcgggc 720

agtttactgt tcggtggcgc agctaacgca ttaaacattc cgcctgggga gtacggtcgc 780

aagattaaaa ctcaaaggaa ttgacggggg cccgcacaag cggtggagca tgtggtttaa 840

ttcgaagcaa cgcgcagaac cttaccagcc cttgacatcc cgatcgcgga ttacggagac 900

gttttccttc agttcggctg gatcggagac aggtgctgca tggctgtcgt cagctcgtgt 960

cgtgagatgt tgggttaagt cccgcaacga gcgcaaccct cgcccttagt tgccagcatt 1020

tagttgggca ctctaagggg actgccgg 1048

<210> 6
<211> 18
<212> DNA
<213> Artificial Sequence

<220>
<223> primer REP 1R


<220>
<221> modified_base
<222> (1)..(4)
<223> I

<220>
<221> modified_base
<222> (7)..(7)
<223> I

<220>
<221> modified_base
<222> (10)..(10)
<223> I

<220>
<221> modified_base

<222>  (15)..(15)
<223>  I

<400>  6
nnnncgncgn catcnggc                                                    18


<210>  7
<211>  18
<212>  DNA
<213>  Artificial Sequence

<220>
<223>  primer Rep2I


<220>
<221>  modified_base
<222>  (1)..(1)
<223>  I

<220>
<221>  modified_base
<222>  (4)..(4)
<223>  I

<220>
<221>  modified_base
<222>  (11)..(11)
<223>  I

<400>  7
ncgncttatc nggcctac                                                    18


<210>  8
<211>  20
<212>  DNA
<213>  Artificial Sequence

<220>
<223>  primer ERIC 1

<400>  8
agtaagtgac tggggtgagc                                                  20


<210>  9
<211>  22
<212>  DNA
<213>  Artificial Sequence

<220>
<223>  primer ERIC 2

<400>  9
atgtaagctc ctggggattc ac                                               22

## Description

### FIELD OF THE INVENTION

The present invention is comprised in the field of biological control of nematodes that are able to infect plants. Specifically, the invention relates to bacteria from the species *Ensifer adhaerens* with nematicidal activity, as well as to the use of said bacteria in methods for biologically controlling nematodes.

### BACKGROUND OF THE INVENTION

Phytoparasitic nematodes are naturally pathogens, but their interactions with other agents causing diseases make it difficult to measure their real impact on crop yield and to make a large-scale estimate. Phytoparasitic nematodes generally cause yearly losses of between 11 and 14% in economically important crops such as legume, cereal, banana tree, yucca, coconut tree, sugar beet, sugar cane, potato, vegetable, ornamental and fruit crops.

The nematode feeding process can cause a reaction in affected plant cells, resulting in root tip and terminal bud death or weakening, lesion formation and torn tissues, bulges and galls, and stem and leaf wrinkling and deformation. Some of these manifestations are caused by decomposition of the tissue affected by nematode enzymes, which, with or without the aid of toxic metabolites, causes tissue disintegration and cell death. Other symptoms, such as galling caused by nematodes from the genus *Meloidogyne,* are caused by abnormal cell elongation (hypertrophy), by cell division suppression or by controlled cell division process stimulation resulting in the formation of galls (hyperplasia) or of a large number of lateral roots in or close to infection sites.

In some cases, however, symptoms are brought about by biochemical interactions of the plants with the nematodes, affecting general plant physiology, as well as the role nematodes play in forming wounds through which other pathogens, which are primarily responsible for the damage caused, penetrate said plants.

With the application of the new European Directive 2009/128/EC for the sustainable use of phytosanitary products, most of the chemicals intended for controlling these plant parasites and pathogens are being withdrawn due to their high toxicity and treatment aggressiveness.

An ecological alternative for treating and controlling phytopathogenic nematodes consists of biologically controlling such nematodes by means of the use of microorganisms with nematicidal activity. In this sense, the use of some bacteria strains belonging to the species *Sinorhizobium meliloti* has been described. Particularly, Shahid Shaukat et al. (Shahid Shaukat S. et al. 2002. Pakistan Journal of Biol. Sci., 5(6): 669-671) disclosed the *in vitro* nematicidal activity of *Sinorhizobium meliloti* against *Meloidogyne incognita* juveniles. However, said nematicidal activity is not present in other species pertaining to the genus *Sinorhizobium* as evidenced by Duponnois et al. (Duponnois R. et al. 1999. Eur. J. Soil. Biol., 35(2):99-105) that discloses that strains of the species *Sinorhizobium terangae* have no effect on the multiplication of *Meloidogyne javanica* on the plant *Acacia holosericea.*

Therefore, there is a need for new ecological alternatives for biologically controlling phytopathogenic nematodes.

### SUMMARY OF THE INVENTION

In a first aspect, the invention relates to a microorganism from the species *Ensifer adhaerens* having nematicidal activity or a mutant of said microorganism maintaining said nematicidal activity selected from the group consisting of:
(i) *E. adhaerens* strain B742, deposited in the Spanish Type Culture Collection (CECT) with accession number CECT 8809,
(ii) *E. adhaerens* strain B758, deposited in the Spanish Type Culture Collection (CECT) with accession number CECT 8810, and
(iii) *E. adhaerens* strain B760, deposited in the Spanish Type Culture Collection (CECT) with accession number CECT 8808.

In a second aspect, the invention relates to a biologically pure culture of a microorganism according to the first aspect.

In a third aspect, the invention relates to a method for obtaining a biomass of the microorganism according to the first aspect comprising culturing said microorganism under conditions suitable for growth.

In a fourth aspect, the invention relates to a biomass of the microorganism according to the first aspect obtainable by means of the method of the third aspect.

In a fifth aspect, the invention relates to a phytosanitary product comprising a microorganism from the species *Ensifer adhaerens* having nematicidal activity or a mutant of said microorganism maintaining said nematicidal activity, or a biologically pure culture of said microorganism or mutant thereof, or a biomass of said microorganism or mutant thereof, and an agriculturally acceptable excipient.

In a sixth aspect, the invention relates to a supplemented seed comprising a seed, and furthermore a microorganism from the species *Ensifer adhaerens* having nematicidal activity or a mutant of said microorganism maintaining said nematicidal activity, or a biologically pure culture of said microorganism or mutant thereof, or a biomass of said microorganism or mutant thereof, or a phytosanitary product according to the fifth aspect.

In a seventh aspect, the invention relates to a method for biologically controlling a nematode comprising applying to said nematode a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to the fifth aspect.

In an eighth aspect, the invention relates to a method for preventing plant infection caused by a nematode comprising applying an effective amount of a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to the fifth aspect, on said plant, on the seed of said plant, in the soil surrounding said plant or on a nematode susceptible of infecting said plant, or alternatively planting a seed of said plant supplemented with a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to the fifth aspect.

In a ninth aspect, the invention relates to a method for treating a plant infected by a nematode comprising applying an effective amount of a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to the fifth aspect, on said plant or in the soil surrounding said plant.

### BRIEF DESCRIPTION OF THE DRAWINGS

Fig. 1. Morphological characteristics of E. *adhaerens* strains B742, B758 and B760 grown at 26°C in nutritive agar plates.
Fig. 2. Morphological characteristics of E. *adhaerens* strains B742, B758 and B760 grown at 37°C in nutritive agar plates.
Fig. 3. Differentiation at strain level of E. *adhaerens* B742, B758, B760 and DSMZ 23677. Neg, negative control.
Fig. 4. Cumulative hatching of *M. javanica* eggs incubated with E. *adhaerens* strain B758 for 21 days compared to untreated control (control) and chemical control (fenamiphos). Values are the mean of eight replications per treatment.
Fig. 5. *In vitro* efficacy of E. *adhaerens* strain B758 and the reference chemical control (fenamiphos) against *M. javanica* eggs corrected with respect to the control. Values are the mean of eight replications per treatment. The different letters indicate statistically significant differences.
Fig. 6. Reproduction factor of treatments with E. *adhaerens* strain B758 and the reference chemical control (fenamiphos) compared to control in tomato plants. Values are the mean of ten replications per treatment. The different letters indicate statistically significant differences.
Fig. 7. *In vivo* nematicidal activity of E. *adhaerens* strain B758 and the reference chemical control (fenamiphos) against eggs and juveniles of *M. javanica* in tomato plants corrected with respect to the control. Values are the mean of ten replications per treatment. The different letters indicate statistically significant differences.
Fig. 8. Cumulative hatching of *M. javanica* eggs incubated with E. *adhaerens* strains B742, B758, B760 and DSMZ 23677 for 21 days compared to untreated control (control) and chemical control (fenamiphos).
Fig. 9. *In vitro* efficacy of E. *adhaerens* strains B742, B758, B760 and DSMZ 23677 and the reference chemical control (fenamiphos) against eggs of *M.javanica* corrected with respect to the control.

### DETAILED DESCRIPTION OF THE INVENTION

The inventors of the present invention have surprisingly found that the bacterial species *Ensifer adhaerens* have nematicidal activity. Specifically, the inventors have isolated three bacterial strains of *Ensifer adhaerens* (*E. adhaerens* strains B742, B758 and B760) (Example 1) and have observed that said strains are able to inhibit nematode egg hatching from the species *Meloidogyne javanica* in *in vitro* assays (Example 4). Said nematicidal activity was also confirmed *in vivo* on tomato plants of the "Marmande" cultivar for E. *adhaerens* strain B758 (Example 3). The inventors have also discovered that other members of the species E. *adhaerens* such as strain with deposit number DSMZ 23677 have nematicidal activity (Example 4).

The following inventive aspects have been developed based on these discoveries.

### Microorganisms and culture

In a first aspect, the invention relates to a microorganism from the species *Ensifer adhaerens* having nematicidal activity or a mutant of said microorganism maintaining said nematicidal activity, hereinafter referred to as microorganisms of the invention, wherein said microorganism is selected from the group consisting of:
(i) *E. adhaerens* strain B742, deposited in the Spanish Type Culture Collection (CECT) with accession number CECT 8809,
(ii) *E. adhaerens* strain B758, deposited in the Spanish Type Culture Collection (CECT) with accession number CECT 8810, and
(iii) *E. adhaerens* strain B760, deposited in the Spanish Type Culture Collection (CECT) with accession number CECT 8808.

Said strains were deposited before the date of filing the present patent application in the Spanish Type Culture Collection (CECT), as a legally recognized depositary institution for that purpose in accordance with the Budapest Treaty of April 28, 1977, on international recognition of the deposit of microorganisms for the purposes of patent.

The depositor was Futureco Bioscience, S.A. with registered office at Avda. Cadi, nave 19-23, P. I. Sant Pere Molanta, 08799, Olèrdola, Barcelona, Spain.

The morphological and molecular characteristics of said strains are shown in Example 1, and the culture conditions are described below in the context of the method for obtaining biomass of the invention.

The microorganisms of the invention belong to a species identified in the NCBI database by Taxonomy ID: 106592. The species *Ensifer adhaerens* was described by Casida, 1982 (Casida Jr., L.E. (1982). Ensifer adhaerens gen. nov., sp. nov.: a bacterial predator of bacteria in soil. Int. J. Syst. Bacteriol., 32:339-345). *Sinorhizobium morelense* and *Sinorhizobium adhaerens* are synonims of *Ensifer adhaerens.*

The term "microorganisms of the invention" includes E. *adhaerens* strains B742 (CECT 8809), B758 (CECT 8810), B760 (CECT 8808) with nematicidal activity and also their mutants maintaining said nematicidal activity. In a particular embodiment, the microorganism of the invention is E. *adhaerens* strain B742, deposited in the Spanish Type Culture Collection (CECT) with accession number CECT 8809 or a mutant thereof. In another particular embodiment, the microorganism of the invention is E. *adhaerens* strain B760, deposited in the Spanish Type Culture Collection (CECT) with accession number CECT 8808 or a mutant thereof. In a preferred embodiment, the microorganism of the invention is E. *adhaerens* strain B758, deposited in the Spanish Type Culture Collection (CECT) with accession number CECT 8810 or a mutant thereof.

As it is used in the first aspect of the invention, the term "mutant" refers to any microorganism resulting from a mutation or change in the DNA of one or several genes of E. *adhaerens* strains B742, B758 or B760 (CECT 8809, CECT 8810 or CECT 8808, respectively) maintaining essentially the same characteristics as the parent strain, and specifically essentially maintaining the nematicidal activity.

As it is used herein, the expression "nematicidal activity" refers to the ability to inhibit egg hatching and/or paralyze larval forms of a nematode in any of their stages or to prevent a nematode from developing or growing.

As it is used herein, the term "nematode" refers to a phylum of pluricellular pseudocoelomate organisms of the group Ecdysozoa. As it is used herein, the term "nematode" includes any nematode. Although the microorganisms of the invention can have nematicidal activity on any nematode, they preferably have nematicidal activity on a phytoparasitic or phytopathogenic nematode.

As it is used herein, the term "plant-parasitic nematode" or "plant-pathogenic nematode" or "phytoparasitic nematode" or "phytopathogenic nematode" refers to a nematode with the ability to parasite a plant organism or to cause a disease in a plant organism, including:
- *Migratory endoparasites:* motile nematodes feeding inside plant tissue. All the stages of the life cycle are motile except the egg. They perforate plant tissue, moving from cell to cell. While they feed, they deposit eggs in cortical tissue of the plant or in the soil surrounding the root. Damaged cells release toxins which cause the death of adjacent cells, giving rise to necrotic tissue spots. Pathogenic bacteria and fungi frequently enter through the damaged zones causing rot in the roots. Examples: *Pratylenchus* (*P. vulnus, P. penetrans, P. brachyurus, P. coffeae, P. goodeyi, P. zeae, P. thornei, P. neglectus), Radopholus (R. similis, R. citrophilus), Scutellonema bradys, Hirschmanniella.*
- *Sedentary endoparasites:* the adult penetrates the plant and attaches itself to it, leaving part of its body exposed to the exterior. They generally feed on syncytia. Examples: *Meloidogyne (M. incognita, M. javanica, M. arenaria, M*. *hapla), Globodera (G. pallida, G. rostochiensis), Heterodera (H. schachtii, H. trifolii, H. goettingiana, H. avenae).*
- *Ectoparasites:* free-living phytophagous nematodes. They only introduce the stylet, which can be very weak (*Tylenchus,* citrus nematode), in the root affecting only root hairs, or very long (*Longidorus* and *Xiphinema*), allowing them to feed off deep tissue cells. These latter species are very problematic on an agronomical level because they are virus-transmitting nematodes.

The microorganisms of the invention can have nematicidal activity on any plant-parasitic or -pathogenic nematode of those mentioned above. Illustrative, non-limiting examples of plant-parasitic or plant-pathogenic nematodes on which the microorganisms of the invention can have nematicidal activity include nematodes belonging to the genera *Meloidogyne, Heterodera, Globodera, Pratylenchus, Paratylenchus, Rotylenchus, Xiphinema, Trichodorus, Ditylenchus, Criconemella (Mesocriconema), Helicotylechus, Longidorus, Paratrichodorus, Belonolaimus* and *Radopholus.*

In a particular embodiment, the microorganisms of the invention have nematicidal activity on a nematode selected from the genera *Meloidogyne, Globodera, Heterodera, Pratylenchus, Tylenchulus, Radopholus* and *Xiphinema.* In an even more particular embodiment, the microorganisms of the invention have nematicidal activity on a nematode from the genus *Meloidogyne.* In a still more particular embodiment, the microorganisms of the invention have nematicidal activity on *Meloidogyne javanica.* In another particular embodiment, the microorganisms of the invention have nematicidal activity on a nematode from the genus *Globodera.*

As it is used herein, the term *"Meloidogyne"* refers to a genus of nematodes identified in the NCBI database by Taxonomy ID: 189290.

As it is used herein, the term *"Meloidogyne javanica"* or *"M. javanica"* refers to a species of the so-called root-knot nematodes identified in the NCBI database by Taxonomy ID: 6303. Root-knot nematodes are plant-parasitic nematodes from the genus *Meloidogyne* infecting plant roots giving rise to the development of galls in root knots which drain out the plant nutrients.

As it is used herein, the term *"Globodera"* refers to a genus of nematodes identified in the NCBI database by Taxonomy ID: 31242 and also called "cyst-forming nematodes".

A nematode life cycle includes 6 stages, including the egg, 4 juvenile or larval stages and one adult stage. All stages, with the exception of the first juvenile that is formed from the egg, are preceded by a molt.

The microorganisms of the invention can have nematicidal activity on any of the stages of a nematode, including eggs, juvenile forms and adult forms. In a particular embodiment, the microorganisms of the invention have nematicidal activity on eggs of nematodes. In another particular embodiment, the microorganisms of the invention have nematicidal activity on juvenile forms of nematodes. In a particular embodiment, the microorganisms of the invention have nematicidal activity on eggs of nematodes and on juvenile forms of nematodes. In another particular embodiment, the microorganisms of the invention have nematicidal activity on the adult form of nematodes. In another particular embodiment, the microorganisms of the invention have nematicidal activity on eggs of nematodes and on the adult form of nematodes. In another particular embodiment, the microorganisms of the invention have nematicidal activity on juvenile forms of nematodes and on the adult form of nematodes. In another particular embodiment, the microorganisms of the invention have nematicidal activity on eggs of nematodes, on juvenile forms of nematodes and on the adult form of nematodes.

The nematicidal activity of a microorganism can be determined by the person skilled in the art by means of any *in vivo* or *in vitro* assay that allows determining and/or quantifying the ability of a microorganism to interfere with nematode development in any of its stages. Illustrative examples of said assays are the following:
- An *in vitro* assay on eggs in which, after an incubation period of time of the eggs of nematodes under conditions suitable for hatching, the percentage of eggs that hatched in the presence of the control agent, in this case the microorganism whose eventual nematicidal activity is to be known, compared with a control sample (for example, the same amount of eggs of nematodes in the absence of the control agent), is determined. By means of such assay the "nematicidal efficacy" parameter can be determined as the difference in the percentage of eggs that hatched in the presence of the microorganism with respect to the control in a specific time period. Any significant reduction in the percentage of eggs that hatched in the presence of the microorganism with respect to the control indicates that said microorganism has nematicidal activity.
- An *in vitro* assay on juveniles in which, after an incubation period of time of juvenile forms of nematodes under conditions suitable for development, the percentage of live juveniles in the presence of the control agent, in this case the microorganism whose eventual nematicidal activity is to be known, compared with a control sample (for example, the same amount of juveniles in the absence of the control agent), is determined. The "nematicidal efficacy" determined by means of such assay is defined as the percentage of mortality of juveniles corrected with respect to the control in a specific time period. Any significant reduction in the percentage of live juveniles in the presence of the microorganism with respect to the control indicates that said microorganism has nematicidal activity.
- An *in vivo* assay in which a plant is inoculated with juvenile forms of the nematode, and after a time period under conditions suitable for plant growth and after being treated with the microorganism whose eventual nematicidal activity is to be known, the number of eggs, and/or juveniles and/or adults of the nematode present in the plant treated with the microorganism compared with the control (for example, said plant untreated with the microorganism) is evaluated. The "nematicidal efficacy" determined by means of such assay is defined as the difference in the percentage of eggs and/or juvenile and/or adult stages in the presence of the microorganism corrected with respect to the control in a specific time period. Any significant reduction in the percentage of eggs and/or juveniles and/or adults in the presence of the microorganism with respect to the control indicates that said microorganism has nematicidal activity.
- An *in vivo* assay in which a plant is inoculated with juvenile forms of the nematode, and after a time period under conditions suitable for plant growth and after being treated with the microorganism whose eventual nematicidal activity is to be known, the number of eggs of the nematode present in the plant treated with the microorganism compared to the initial number of eggs before the treatment is evaluated. The reproduction factor" (RF) determined by means of such assay is defined as the density of final nematode population (Pf) relative to the initial inoculum level (Pi) and is calculated as the final number of eggs/initial number of eggs. This parameter has been widely used in nematological studies to define resistance and susceptibility of plants to plant parasitic nematodes. Any significant reduction in the reproduction factor in the presence of the microorganism indicates that said microorganism has nematicidal activity. Other parameters such as fertility and infectivity are also common parameters used to evaluate the nematicidal activity. Fertility is calculated as the total eggs/ egg mass and the infectivity is calculated as the total egg mass/ plant.

In a particular embodiment, the nematicidal activity of the microorganisms of the invention is determined by means of the assays used in Examples 2 to 4 of the present specification.

The microorganisms of the invention have a nematicidal efficacy of at least 2%; at least 5%; at least 10%; at least 15%; at least 20%; at least 25%; at least 30%; at least 40%; at least 50%; at least 60%; at least 70%; at least 80%; at least 90%; at least 95%; at least 99% or 100% with respect to a control.

In a particular embodiment, the microorganisms of the invention have a nematicidal efficacy, measured as efficacy corrected in the reduction of *M. javanica* egg hatching, determined by means of an *in vitro* assay on *M. javanica* eggs using the conditions described in Example 1 of the specification of at least 60% with respect to a control; or of at least 30% with respect to a control, using the conditions described in Example 4 of the specification.

In a second aspect, the invention relates to a biologically pure culture of a microorganism of the invention.

As it is used in the context of the first aspect of the invention, the expression "biologically pure culture" refers to a culture in which the microorganism of the invention can be found in a proportion of 95% or higher, for example 96% or higher, 97% or higher, 98% or higher, 99% or higher, or 100%, compared with other organisms present in the culture. In a particular embodiment, the biologically pure culture is a biologically pure culture of E. *adhaerens* strain B742 or a mutant thereof. In another particular embodiment, the biologically pure culture is a biologically pure culture of E. *adhaerens* strain B760 or a mutant thereof. In a preferred embodiment, the biologically pure culture is a biologically pure culture of E. *adhaerens* strain B758 or a mutant thereof.

As it is used in the context of the first aspect of the invention, the term "culture" refers to a population of the microorganism of the invention. A culture can comprise other elements in addition to the microorganism of the invention, such as the culture medium or any other substance that can be added to the culture medium that is beneficial for culture growth or maintenance. The term "culture medium" or "medium" is recognized in the art and generally refers to any substance or preparation that is used for the culture of live cells. As it is used in reference to a cell culture, the term "medium" includes the components of the environment surrounding the cells. The medium can be solid medium, liquid medium, gaseous medium, or a mixture of the phases and materials. The growth media include liquid culture media as well as liquid media that do not support cell growth. The medium also includes gelatinous media such as agar, agarose, gelatin and collagen matrices. Exemplary gaseous media include the gas phase to which the cells growing in a Petri dish or another solid or semisolid support are exposed. The term "medium" also refers to a material that must be used in a cell culture, even if it has still not been contacted with the cells. In other words, a liquid rich in nutrients prepared for bacterial culture is a medium. Likewise, a powder mixture which when mixed with water or with another liquid becomes suitable for the cell culture can be called a "powder medium". "Defined medium" refers to the media made up of components having a defined chemical constitution (generally purified). The defined media do not contain biological extracts that are not completely characterized, such as meat broth and yeast extract. "Rich medium" includes media designed to support growth of most or all viable forms of a particular species. Enriched media often include complex biological extracts. Any conventional culture medium suitable for E. *adhaerens* known in the art can be used in the present invention, such as, for example, nutrient broth made up of meat extract (3 g/L) and soy peptone (5 g/L); or LB medium made up of yeast extract (5g/L), tryptone (10g/L) and sodium chloride (10g/L). In a particular embodiment, the culture medium that can be part of the biologically pure culture of the invention is made up of: soy peptone (3g/L), tryptone (17 g/L), glucose (2.5g/L), sodium chloride (5g/L) and dipotassium phosphate (2.5g/L).

### Biomass and method for obtaining biomass

The microorganisms of the invention can be used in the form of a biomass of said microorganism multiplied in culture in methods for biologically controlling nematodes, or for preventing or treating plant infection caused by nematodes.

Therefore, in a third aspect the invention relates to a method for obtaining a biomass of one of the microorganisms of the invention, comprising culturing said microorganism under conditions suitable for growth.

As it is used in the context of the third aspect of the invention, the term "biomass" refers to the biological material of living organisms, particularly of the microorganisms of the invention. In a particular embodiment, the microorganism cultured is E. *adhaerens* strain B742 or a mutant thereof. In another particular embodiment, the microorganism cultured is E. *adhaerens* strain B760 or a mutant thereof. In a preferred embodiment, the microorganism cultured is E. *adhaerens* strain B758 or a mutant thereof.

Conditions suitable for growth of the microorganisms of the invention will be those conditions which allow microorganism maintenance and multiplication. In a particular embodiment, said conditions comprise culturing the microorganism of the invention in the presence of a culture medium or substrate containing one or several carbon sources, one or several nitrogen sources and inorganic and organic salts at concentrations suitable for obtaining maximum biomass yields. Said medium or substrate can be solid or liquid. The carbon sources consist of monosaccharides, polysaccharides, cereals or plant extracts. The nitrogen sources comprise plant protein hydrolysates, peptones or free, pure or mixed amino acids. The salts are sulfates or phosphates of elements such as Na, Ca, Mg, Fe, or K. In a particular embodiment, the culture medium or substrate contains between 1 and 5 carbon sources, between 1 and 5 nitrogen sources and between 1 and 10 salts.

In a particular embodiment, the conditions suitable for growth of the microorganisms of the invention comprise culturing the microorganism in the culture medium under constant temperature, pH and oxygenation conditions. In a particular embodiment, the temperature is comprised between 25°C and 35°C, preferably 30°C. In a particular embodiment, the pH is comprised between 5.0 and 9.0, preferably between 6.0 and 8.0, more preferably 7.0. In a particular embodiment, oxygenation is achieved by means of stirring at speeds between 200 and 500 rpm and/or supplying sterile air at a fixed flow rate between 0.5 and 1.5 vvm; preferably at speeds between 200 and 300 rpm and/or supplying sterile air at a fixed flow rate between 0.8 and 1.2 vvm; more preferably, oxygenation is achieved by means of a variable stirring to maintain a minimum concentration of dissolved oxygen of 80% for 48 hours.

The time during which the microorganism must be kept under conditions suitable for growth is the time needed for the microorganism to reach a concentration corresponding to a minimum substrate to biomass conversion of 80%. In a particular embodiment, said time of growth for reaching these yields is between 1 and 10 days.

Once the microorganism growth step has ended, the obtained biomass can be recovered from the used up substrate by applying one or several unit operations which can comprise centrifugation, decantation, filtration or a combination of several of these operations. Therefore, in a particular embodiment, the method for obtaining the biomass of the microorganisms of the invention additionally comprises a step for separating the biomass from the substrate. In an even more particular embodiment, said biomass is separated from the substrate by means of one or more steps of centrifugation, decantation or filtration, or a combination thereof.

In a particular embodiment, after separating the biomass from the substrate the biomass can be dried using low temperature and/or pressure conditions to remove moisture from the biomass. In a more particular embodiment, said conditions are between 18 and 25°C and between 2 and 6 mm Hg.

A biomass of one of the microorganisms of the invention is obtained by means of the method of the third aspect. Therefore, in a fourth aspect, the invention relates to a biomass of the microorganism of the invention obtainable by means of the method according to the third aspect.

### Phytosanitary product and supplemented seed

In a fifth aspect, the invention relates to a phytosanitary product, hereinafter phytosanitary product of the invention, comprising a microorganism from the species *Ensifer adhaerens* having nematicidal activity or a mutant of said microorganism maintaining said nematicidal activity, or a biologically pure culture of said microorganism or mutant thereof, or a biomass of said microorganism or mutant thereof, and an agriculturally acceptable excipient.

As it is used herein, the term "phytosanitary product" refers to all the substances intended for protecting crops and controlling pests and diseases thereof.

As it is used herein, the term "agriculturally acceptable excipient" refers to any substance or combination of substances which can be used in the agricultural sector, and includes any agriculturally acceptable liquid or solid material which can be added to and/or mixed with the microorganism from the species *Ensifer adhaerens* having nematicidal activity, biologically pure culture or biomass of said microorganism, in order to have it in a simpler or improved form of application, or with an applicable or desirable activation intensity. Due to the nature of the active ingredient of the agricultural composition (microorganism from the species *Ensifer adhaerens* having nematicidal activity), said agriculturally acceptable vehicle has to allow, or not jeopardize or compromise, the viability and the controlling ability of said microorganism.

The authors of the present invention have demonstrated that nematicidal activity is not exclusive of the strains of *Ensifer adhaerens* of the invention and that this nematicidal activity is present in other strains of the species *Ensifer adhaerens.*

Therefore, the phytosanitary product of the invention comprises any microorganism pertaining to the species *Ensifer adhaerens* having nematicidal activity or a mutant of said microorganism maintaining said nematicidal activity, or a biologically pure culture of said microorganism or mutant thereof, or a biomass of said microorganism or mutant thereof as a technical grade active ingredient (TGAI) with nematicidal activity. In a preferred embodiment the microorganism is E. *adhaerens* strain B742. In another preferred embodiment the microorganism is E. *adhaerens* strain B760. In a more preferred embodiment the microorganism is *E*. *adhaerens* strain B758. In another embodiment the microorganism is *E*. *adhaerens* strain TA12-B, deposited in the German Collection of Microorganisms and Cell Cultures (DSMZ) with accession number DSMZ 23677. In another embodiment the phytosanitary product of the invention comprises a combination of two or more of E. *adhaerens* strain B742, *E*. *adhaerens* strain B760, *E. adhaerens* strain B758 and E. *adhaerens* strain TA12-B.

In an embodiment, the phytosanitary product of the invention comprises a microorganism from the species *Ensifer adhaerens* having nematicidal activity or a mutant of said microorganism maintaining said nematicidal activity.

As it is used in the fifth aspect of the invention, the term "mutant" refers to any microorganism resulting from a mutation or change in the DNA of one or several genes of E. *adhaerens* maintaining essentially the same characteristics as the parent strain, and specifically essentially maintaining the nematicidal activity.

The terms "nematicidal activity" and "nematode" have been defined in the context of the first aspect of the invention. All the embodiments disclosed in the first aspect of the invention are applicable to the fifth aspect of the invention.

In another embodiment, the phytosanitary product of the invention comprises a biologically pure culture of a microorganism from the species *Ensifer adhaerens* having nematicidal activity or a mutant of said microorganism maintaining said nematicidal activity.

As it is used in the context of the fifth aspect of the invention, the expression "biologically pure culture" refers to a culture in which the microorganism from the species *Ensifer adhaerens* having nematicidal activity or a mutant of said microorganism maintaining said nematicidal activity can be found in a proportion of 95% or higher, for example 96% or higher, 97% or higher, 98% or higher, 99% or higher, or 100%, compared with other organisms present in the culture. In a particular embodiment, the biologically pure culture is a biologically pure culture of *E*. *adhaerens* strain B742 or a mutant thereof. In another particular embodiment, the biologically pure culture is a biologically pure culture of *E*. *adhaerens* strain B760 or a mutant thereof. In another particular embodiment the biologically pure culture is a biologically pure culture of E. *adhaerens* strain TA12-B, deposited in the German Collection of Microorganisms and Cell Cultures (DSMZ) with accession number DSMZ 23677 or a mutant thereof. In a particular embodiment, the biologically pure culture is a biologically pure culture of E. *adhaerens* strain B758 or a mutant thereof.

As it is used in the context of the fifth aspect of the invention, the term "culture" refers to a population of the microorganism from the species *Ensifer adhaerens* having nematicidal activity or a mutant of said microorganism maintaining said nematicidal activity. A culture can comprise other elements in addition to said microorganism. The term "culture medium" has been defined in the context of the second aspect of the invention. All the embodiments disclosed in the context of the second aspect of the invention are applicable to the fifth aspect of the invention.

In another embodiment, the phytosanitary product of the invention comprises a biomass of a microorganism from the species *Ensifer adhaerens* having nematicidal activity or a mutant of said microorganism maintaining said nematicidal activity.

As it is used in the context of the fifth aspect of the invention, the term "biomass" refers to the biological material of living organisms, particularly of a microorganism from the species *Ensifer adhaerens* having nematicidal activity or a mutant of said microorganism maintaining said nematicidal activity. In a particular embodiment, the microorganism cultured is E. *adhaerens* strain B742 or a mutant thereof. In another particular embodiment, the microorganism cultured is E. *adhaerens* strain B760 or a mutant thereof. In another particular embodiment, the microorganism cultured is E. *adhaerens* strain TA12-B, deposited in the German Collection of Microorganisms and Cell Cultures (DSMZ) with accession number DSMZ 23677 or a mutant thereof. In a preferred embodiment, the microorganism cultured is *E*. *adhaerens* strain B758 or a mutant thereof.

Conditions suitable for growth of *Ensifer adhaerens* and for obtaining biomass of the same have been described in the context of the third aspect of the invention. All the embodiments disclosed in the context of the third aspect of the invention are applicable to the fifth aspect of the invention.

Said phytosanitary product of the invention corresponds to one or several types of phytosanitary product formulations currently described in the FAO/WHO manual in solid or liquid state comprising wettable powders (WP), oily dispersions (OD), encapsulated granules (CG), capsule suspensions (CS), emulsifiable concentrates or granules (EC/EG), granules (GR), microgranules (MG), microemulsions (ME), water-dispersible granules (WG).

The formulation consists of a suitable amount of biomass of *Ensifer adhaerens* to assure a concentration between 10³-10¹⁵ CFU/g product, mixed with organic or inorganic substances, known as excipients, giving it the technical characteristics necessary for complying with FAO/WHO (phytosanitary or nutritional) standards, and with a solvent or carrier for adjusting the concentration. The excipients comprise ionic or anionic substances with surfactant activity, dispersing activity, wetting activity, drying activity and anti-caking activity, among others. They consist of alkyl-sulfuric esters, alkyl aril sulfonates, alkyl aril ethers, ethoxylated alcohols, ethoxylated fatty acids, polyethylene glycol ethers, acid sugars, potassium salts of fatty acids, sodium salts of fatty acids, carrageenin, carboxymethyl cellulose, xanthan gum, lignosulfonic acid, calcium, magnesium and zinc lignosulfonates, silicon oxide, bentonites, glycerin, magnesium, calcium, aluminum, and iron oxides, among others. The formulation in solid state uses as a carrier or solvent one or several substances such as serite, kaolins, diatomaceous earth, acid earth, bran, monosodium glutamate, glucose, dextrose, lactose, sucrose and the like. The formulation in liquid state uses as a carrier or solvent one or several substances such as vegetable oils, light paraffin oils, polyethylene glycol esters with saturated or unsaturated fatty acids, vegetable glycerin and the like.

The phytosanitary product of the invention can furthermore contain, if desired, other ingredients or constituents commonly used in phytosanitary products such as, but not limited to, solvents, active agents or pH regulators, fertilizers, etc., provided that they all allow or do not jeopardize or compromise viability of the microorganism present in the phytosanitary product. Said ingredients or constituents commonly used in phytosanitary products are generally known by the persons skilled in the art.

The phytosanitary product of the invention can be obtained by conventional methods generally based on mixing suitable amounts of the different components of the agricultural composition.

In a sixth aspect, the invention relates to a supplemented seed, hereinafter supplemented seed of the invention, comprising a seed, and furthermore a microorganism from the species *Ensifer adhaerens* having nematicidal activity or a mutant of said microorganism maintaining said nematicidal activity, or a biologically pure culture of said microorganism or mutant thereof, or a biomass of said microorganism or mutant thereof, or the phytosanitary product of the invention. Said seed can be a seed of any plant of interest in agriculture, forestry, food (for human or animal food), ornamental applications, energetic applications, etc.

In a particular embodiment, the supplemented seed of the invention comprises a seed of a plant of a family selected from *Solanaceae, Fabaceae, Malvaceae, Amaranthaceae* and *Cucurbitaceae.*

As it is used herein, the term *"Solanaceae"* refers to a family of plants identified in the NCBI database by Taxonomy ID: 4070. It is a family of herbaceous or woody plants with simple, alternate leaves and without stipules, belonging to the order Solanales, of the dicotyledons. Illustrative, non-limiting examples of plants from the family *Solanaceae* include the genera *Solanum, Lycianthes, Cestrum, Nolana, Lycium, Nicotiana* and *Brunfelsia.*

In a more particular embodiment, the supplemented seed of the invention comprises a seed of a plant from the family *Solanaceae,* preferably from the genus *Solanum.*

As it is used herein, the term *"Solanum"* refers to a genus of plants identified in the NCBI database by Taxonomy ID: 4107. Illustrative, non-limiting examples of plants from the genus *Solanum* include *S. lycopersicum* (tomato), *S. tuberosum* (potato) and *S. melongena* (eggplant).

In an even more particular embodiment, the supplemented seed of the invention comprises a seed of a plant from the species *Solanum lycopersicum.*

As it is used herein, the term *"Solanum lycopersicum"* refers to a species of plants identified in the NCBI database by Taxonomy ID: 4081, commonly known as the tomato. The term *"Solanum lycopersicum"* includes any tomato variety, including by way of illustration *S. lycopersicum var. cerasiforme* (cherry tomato).

In a still more particular embodiment, the supplemented seed of the invention comprises a seed of a plant from the species *Solanum lycopersicum* "Marmande" variety.

The "Marmande" variety is an extremely early tomato variety. The plant has a high stem and the number of fruits per floral level ranges from 5 to 10; said fruits are large, flattened and ribbed, and have a bright red color.

As it is used herein, the term *"Fabaceae"* refers to a family of plants, commonly known as legumes, identified in the NCBI database by Taxonomy ID: 3803. In a particular embodiment, the plant belongs to the genus *Glycine.* In an even more particular embodiment, the plant belongs to the plant species *Glycine max* (Taxonomy ID: 3847) which produces soybean.

As it is used herein, the term *"Malvaceae"* refers to a family of plants identified in the NCBI database by Taxonomy ID: 3629. In a particular embodiment, the plant belongs to the genus *Gossypium* (Taxonomy ID: 3847), to which different species producing cotton belong.

As it is used herein, the term *"Amaranthaceae"* refers to a family of plants identified in the NCBI database by Taxonomy ID: 3563. In a particular embodiment, the plant belongs to the genus *Beta,* more specifically to the plant species *Beta vulgaris* (Taxonomy ID: 161934) which produces beet.

As it is used herein, the term *"Cucurbitaceae"* refers to a family of plants identified in the NCBI database by Taxonomy ID: 3650. Illustrative, non-limiting examples of plants from the family *Cucurbitaceae* whose seeds can be used to obtain the supplemented seed of the invention include the genera *Cucurbita* (plants producing pumpkin and zucchini), *Lagenaria, Citrullus* (plants producing watermelon), *Cucumis* (plants producing cucumber and melon) and *Luffa.* In a particular embodiment, the plant belongs to the family *Cucurbitaceae.*

In an embodiment the supplemented seed comprises a microorganism from the species *Ensifer adhaerens* having nematicidal activity or a mutant of said microorganism maintaining said nematicidal activity. In another embodiment the supplemented seed comprises a biologically pure culture of a microorganism from the species *Ensifer adhaerens* having nematicidal activity or a mutant of said microorganism maintaining said nematicidal activity. In another embodiment the supplemented seed comprises a biomass of a microorganism from the species *Ensifer adhaerens* having nematicidal activity or a mutant of said microorganism maintaining said nematicidal activity. In another embodiment the supplemented seed of the invention comprises the phytosanitary product of the invention. All the embodiments previously disclosed in the context of the first, second, third, fourth and fifth aspects of the invention are applicable to the sixth aspect of the invention.

The microorganism *Ensifer adhaerens* can completely or partially cover the seed.

Said supplemented seed of the invention can be obtained by conventional methods; by way of illustration, it can be obtained by submerging the seed in a pure suspension or culture of the microorganism, or alternatively by spraying said suspension or culture on the seed. Other methods comprise mixing the seed and the microorganism previously incorporated in an inert solid support that facilitates adhesion of the microorganism to said seed.

Furthermore, an agricultural-type adhesive containing a suitable concentration of said microorganism, such as an organic-type polymer or gel, such as acacia gum, alginate polymers, methyl cellulose, etc., can be used to improve adhesion of the microorganism to the seed. Therefore, in a particular embodiment, the supplemented seed of the invention is supported, attached or adhered to an adhesive, such as an agriculturally acceptable adhesive, for example, an organic polymer that is inert for the microorganism (for example, acacia gum, alginate, methyl cellulose, etc.).

### Method for biologically controlling a nematode

In a seventh aspect, the invention relates to a method for biologically controlling a nematode comprising applying to said nematode a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to the fifth aspect of the invention.

As it is used herein, the term "biologically controlling a nematode" refers to the use of living organisms to control the nematode population, i.e., to control their development, reproduction, number of individuals, etc., either directly (by the action of the organisms used as biological control agents) or indirectly (as a result of the compounds produced by said organisms). Biologically controlling nematodes can involve destroying nematodes or preventing nematodes from developing. As it is used herein, biologically controlling nematodes also covers controlling nematode offspring (development of viable cysts and/or egg masses).

The method according to the seventh aspect comprises applying to the nematode to be biologically controlled a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to the fifth aspect of the invention.

The term "nematode" has been previously defined, as have particular and preferred embodiments thereof. In a particular embodiment, the nematode is a plant-parasitic nematode. In a particular embodiment, the nematode is selected from the genera *Meloidogyne, Globodera, Heterodera, Pratylenchus, Tylenchulus, Radophulus* and *Xiphinema.* In an even more particular embodiment, the nematode is selected from the genera *Meloidogyne* and *Globodera.* In a still more particular embodiment, the nematode is *Meloidogyne javanica.*

Contacting the nematode to be biologically controlled with the microorganism, culture, biomass or phytosanitary product of the invention can be carried out by any suitable technique or method, using suitable apparatuses, equipment and devices. The person skilled in the art knows the techniques and methods, as well as suitable apparatuses, equipment and devices for applying biological control agents. The method according to the seventh aspect can be carried out by applying the microorganism, culture, biomass or phytosanitary product directly on the nematode, in the nematode's habitat, for example in the soil where a plant is going to be or is grown, in the plant susceptible of being attacked by said nematode, or on a seed of a plant susceptible of being attacked by said nematode. The microorganism, culture, biomass or phytosanitary product can also be applied by means of manual or automated irrigation systems.

In a particular embodiment, the method according to the seventh aspect additionally comprises the use of a fungicide, nematicide or soil insecticide.

As it is used herein, the term "fungicide" refers to agents with the ability to increase mortality or inhibit growth rate of fungi, and also the ability to inhibit germination of fungi spores. Illustrative, non-limiting examples of fungicides include:
- Copper compounds: copper chloride, copper oxychloride, cupric oxide, "Bordeaux mixture", copper-8 quinolinolate, basic copper carbonate, copper naphthenate, copper sulfate, copper chromate, copper oleate.
- Mercury compounds: Calomel (mercurous chloride), mercuric oxide, mercury lactate, mercuram (phenylmercuric acetate), MEMC (methoxyethyl mercuric chloride), PMA (phenylmercuric acetate).
- Tin compounds: fentin acetate (triphenyltin acetate), fentin chloride (triphenyltin chloride), butyltin oxide, Plictran (trichlorohexyltin hydroxide).
- Zinc compounds: zinc chloride, chromate, naphthenate and oleate.
- Metallic compounds: potassium permanganate, cadmium chloride, ferrous sulfate, neo-asozin (monomethyl ferric arsenate), rhizoctol (methyl arsenic sulfite), urbacid, chrome naphthalene.
- Sulfur compounds: sofril, lime sulfur.
- Organophosphorous compounds: pyrazophos, IBP/kitazin, edifenphos, ditalimfos.
- Dithiocarbamates: zineb, maneb, mancozeb, nabam, thiram, ferbam, bunema, vapam, metiram, methylmetiram.
- Carbamates: thiophanate, thiophanate-methyl.
- Halogenated hydrocarbons: 1,1-dichloromethane, dibromomethane, bromomethane, chloropicrin, carbon tetrachloride, p-dichlorobenzene, hexachlorobenzene, chloroneb, dodecyl ammonium bromide, hexachlorophene, pentachlorophenol, isobac (hexachlorophene monosodium salt), etc.
- Aromatic nitro compounds: dinitrophenol, nitrodiphenyl, DNOC (4,6-dinitro-o-cresol), dinobuton, tecnazene, binapacryl, dinocap, nirit, brassicol (pentachloronitrobenzene), etc.
- Quinines: chloranil, dichlone, benzoquinone, dithianon, etc.
- Anilides: benodanil, pyrocarbolid, carboxin, oxycarboxin, salicylanilide, etc.
- Guanidine compounds: dodine (dodecylguanidine acetate), guazatine, etc.
- Phthalimides: folpet, captan, captafol, chlorothalonil, dimetakion, etc.
- Pyrimidines: dimethirimol, ethirimol, bupirimate, etc.
- Thiodiazoles: dazomet, terrazole, milneb, etc.
- Triazines: anilazine, triadimefon, etc.
- Isoxazolones: hymexazol, drazoxolon, etc.
- Imidazoles: glyodin, bencmil, thiabendazole, triforine, carbendazim, etc.
- Other heterocyclic compounds: tridemorph, chinomethionate, etc.
- Antibiotics: blasticidin, gliotoxin, griseofulvin, polyoxin, phytobacteriomycin, kasugamycin, validamycin, etc.
- Oils: anthracene, ammonium naphthenate, etc.
- Aldehydes, ketones, oxides: formaldehyde, p-formaldehyde, allyl alcohol, ethylene oxide, propylene oxide, etc.
- Others: rhodamine, Trapex® (methyl isocyanate), dichlofuanid, fenaminosulf, etc.
- Biological products: *Trichoderma* (Tusal®), *Streptomyces* (Actinovate®), etc.

As it is used herein, the term "nematicide" refers to agents with the ability to inhibit egg hatching and/or paralyze larval forms in any of their stages and/or adult forms or to prevent nematodes from developing. Illustrative, non-limiting examples of nematicides which can be used in the method for biologically controlling a nematode include:
- Fumigants: D-D (mixture of 1,2-dichloropropane and 1,3-dichloropropene); ethylene dibromide; 1,2-dibromo-3-chloropropane; methyl bromide; chloropicrin (nitrochloroform); metam sodium (sodium methyldithiocarbamate); Dazomet (3,5-dimethyl-1,3,5-thiadiazinan-2-thione); methyl isothiocyanate (MITC); sodium tetrathiocarbonate.
- Carbamates: aldicarb (2-methyl-2-(methylthio)propanal O-(N-methylcarbamoyl)oxime); aldoxicarb ([(E)-(2-methyl-2-methylsulfonylpropylidene)amino] N-methylcarbamate); carbofuran (2,2-dimethyl-2,3-dihydro-1-benzofuran-7-yl methylcarbamate); oxamyl (methyl 2-(dimethylamino)-N-[(methylcarbamoyl)oxy]-2-oxoethanimidothioate).
- Organophosphates: ethoprop (O-ethyl S,S-dipropylphosphorodithioate); fenamiphos (tyl-3-methyl-4-(methylthio)phenyl(1-methylethyl)phosphoramidate)); cadusafos (*O*-ethyl S,S-di-sec-butyl phosphorodithioate); fosthiazate ((*RS)-S-sec-butyl O*-ethyl 2-oxo-1,3-thiazolidin-3-ylphosphonothioate); thionazin (*O,O*-Diethyl *O*-2-pyrazinyl phosphorothioate); fosthietan (diethyl 1,3-dithiethan-2-ylidenephosphoramidate); isazophos (*O*-5-chloro-1-isopropyl-1H-1,2,4-triazol-3-yl *O,O*-diethyl phosphorothioate).
- Biochemical agents: DiTera® (mixture of compounds produced by the nematode-parasitic fungus *Myrothecium verrucaria*), Cladosan® (product obtained from exoskeletons of crab and lobsters, rich in chitin and urea), Sincoin® (mixture of extracts of prickly pear *Opuntia lindheimeri,* oak *Quercus falcata,* sumac *Rhus aromatica,* and mangrove *Rhizophora mangle*)*.*
- Biological products: spores of the fungus *Paecilomyces lilacinus* (BioAct®, Biostat®), *Bacillus firmus* (Flocter®).

As it is used herein, the term "soil insecticide" refers to an agent able to kill, control and/or repel insects the life cycle of which includes a stage in soil. Illustrative, non-limiting examples of soil insecticides include: Aldrin (hexachlorohexahydro-endo-exodimethane naphthalene); Dieldrin (hexachloroepoxy-octahydro-endo-exo-dimethanonaphthalene); Chlorodane (octachloro-4,7-methanotetrahydro-indane); Temik® (Aldicarb: 2-methyl-2-(methylthio)propanal-O-(N-methylcarbamoyl)oxime); Carbofuran (2,3-dihydro-2,2-dimethyl-7-benzofuran methyl carbamate); Landrin® (trimethyl phenyl methylcarbamate); Chlorfenvinphos (2-chloro-1-(2,4-dichlorophenyl)vinyl diethyl phosphate); Phorate® (O,O-diethyl-S-[(ethylthio)methyl] phosphorodithioate); Terburox® (S-tert-butylthiomethyl-O-O-diethyl phosphorodithioate); biological products such as fungus *Beauveria bassiana* (Botanigard®, Naturalis®), spores of the fungus *Paecilomyces fumosoroseus* (NoFly®, PreFeRal®), *Bacillus thuringiensis* (Dipel®) and the like.

All the embodiments defined for the first, second, third, fourth, fifth and sixth aspects of the invention are applicable to the seventh aspect of the invention.

Particularly, in an embodiment the microorganism from the species *Ensifer adhaerens* is E. *adhaerens* strain B742, deposited in the Spanish Type Culture Collection (CECT) with accession number CECT 8809. In another embodiment the microorganism from the species *Ensifer adhaerens* is E. *adhaerens* strain B758, deposited in the Spanish Type Culture Collection (CECT) with accession number CECT 8810. In another embodiment the microorganism from the species *Ensifer adhaerens* is E. *adhaerens* strain B760, deposited in the Spanish Type Culture Collection (CECT) with accession number CECT 8808. In another embodiment the microorganism from the species *Ensifer adhaerens* is E. *adhaerens* strain TA12-B, deposited in the German Collection of Microorganisms and Cell Cultures (DSMZ) with accession number DSMZ 23677. In another embodiment, the microorganism from the species *Ensifer adhaerens* is a combination of two or more of E. *adhaerens* strain B742, *E. adhaerens* strain B758, *E. adhaerens* strain B760 and E. *adhaerens* strain TA12-B.

### Method for preventing plant infection caused by a nematode and method for treating a plant infected by a nematode

In an eighth aspect, the invention relates to a method for preventing plant infection caused by a nematode, comprising applying an effective amount of a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to the fifth aspect, on said plant, on the seed of said plant, in the soil surrounding said plant or on a nematode susceptible of infecting said plant, or alternatively planting a seed of said plant supplemented with a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to the fifth aspect.

As it is used herein, the expression "preventing plant infection caused by a nematode" refers to any activity preventing, impeding, holding up or delaying plant infection caused by a nematode.

The term "nematode" has been previously defined. In a preferred embodiment, the nematode is a plant-parasitic nematode. In a more preferred embodiment, the plant-parasitic nematode is selected from the genera *Meloidogyne, Globodera, Heterodera, Pratylenchus, Tylenchulus* and *Radophulus.* In an even more particular embodiment, the plant-parasitic nematode is from the genus *Meloidogyne.* In a still more particular embodiment, the plant-parasitic nematode is *Meloidogyne javanica.*

As it is used herein, the term "plant" refers to any living being belonging to the kingdom *Plantae (sensu strictissimo)* including eukaryotic living beings with photosynthetic ability, without locomotor ability, and the cell walls of which are mainly made up of cellulose. In *sensu strictissimo,* the term *Plantae* includes the clade *Embrophyta* (land plants: vascular and non-vascular (bryophytes)) comprising: hepaticas, hornwort, moss, lycopodiophyta and plants with seeds (gymnosperms and angiosperms). In the present invention this term refers to species of the taxon *Angiospermae.*

In a particular embodiment, the plant belongs to a family selected from *Solanaceae, Musaceae, Fabaceae, Malvaceae, Amaranthaceae, Vitaceae, Rutaceae, Cucurbitaceae, Poaceae, Rubiaceae,* ornamental plant families or a family of fruit trees.

The terms *"Solanaceae", "Fabaceae", "Malvaceae", "Amaranthaceae", "Cucurbitaceae",* as well as preferred and particular embodiments thereof, have previously been defined.

In a preferred embodiment, the plant belongs to the family *Solanaceae.* In a more preferred embodiment, the plant from the family *Solanaceae* belongs to the genus *Solanum.* In an even more preferred embodiment, the plant from the genus *Solanum* is *Solanum lycopersicum.* In a still more preferred embodiment, the plant from the genus *Solanum* is *Solanum lycopersicum* "Marmande" variety. The terms *"Solanum", "Solanum lycopersicum"* and *"Solanum lycopersicum* "Marmande" variety" have previously been described.

As it is used herein, the term *"Musaceae"* refers to a family of plants identified in the NCBI database by Taxonomy ID: 4637. Illustrative, non-limiting examples of plants from the family *Musaceae* include the genera *Musa* and *Ensete.* In a particular embodiment, the seed belongs to the genus *Musa* (Taxonomy ID: 4640) to which different species producing bananas belong.

As it is used herein, the term *"Vitaceae"* refers to a family of plants identified in the NCBI database by Taxonomy ID: 3602. In a particular embodiment, the plant belongs to the genus *Vitis,* more specifically to the plant species *Vitis vinifera* (Taxonomy ID: 29760) which produces grapes.

As it is used herein, the term *"Rutaceae"* refers to a family of plants identified in the NCBI database by Taxonomy ID: 23513. In a particular embodiment, the plant belongs to the genus *Citrus* (Taxonomy ID: 2706) formed by different plants species producing citrus fruits such as oranges, lemons, grapefruits and limes.

As it is used herein, the term *"Poaceae"* refers to a family of plants identified in the NCBI database by Taxonomy ID: 4479. Illustrative, non-limiting examples of plants of this family are plants producing wheat, barley, rice or corn.

As it is used herein, the term *"Rubiaceae"* refers to a family of plants identified in the NCBI database by Taxonomy ID: 24966. In a particular embodiment, the plants from the family *Rubiaceae* are plants that produce coffee.

As it is used herein, the term "ornamental" refers to plants that are cultured and sold for decorative purposes given their aesthetic characteristics, such as flowers, leaves, aroma, foliage texture, fruits or stems in gardens, an indoor plant or a cut flower. Illustrative, non-limiting examples of families included in this definition are *Liliaceae* (including species of tulips, dracaenas or lilies), *Iridiaceae* (including species from the genera *Crocus, Iris* or *Gladiolus), Amaryllidaceae* (including species such as a daffodil), *Rosaceae* (including species of roses) or *Geraniae* (including species of geraniums).

As it is used herein, the term "fruit tree" refers to any woody tree or vine that produces a fruit of agricultural or industrial interest. The term "fruit" refers to a part of a plant with flowers that is derived from specific tissues of the flower and is not limited to culinary fruits. Illustrative examples of fruit trees whose seeds can be used to obtain the supplemented seed of the invention include avocado, almond tree, banana tree or plantain tree, cherry tree, plum tree, citrus trees, apricot tree, peach tree, durian tree, orange tree, sour cherry tree, mango, apple tree, quince tree, loquat tree, walnut tree and pear tree.

The prevention of plant infection caused by a nematode can be carried out on the plant once it has been planted or on the seed of said plant before it is planted. Therefore, the method according to the eighth aspect comprises the following alternatives:
(i) Applying an effective amount of the microorganism, biologically pure culture, biomass or phytosanitary product on said plant, on the seed of said plant, in the soil surrounding said plant or on a nematode susceptible of infecting said plant.

As it is used herein, the term "effective amount" refers to the amount necessary for stabilizing, impeding, holding up or delaying progression of stages of development of the nematode in question in the plant, and thereby obtaining beneficial or desired results. The term "effective amount" refers to the minimum amount of microorganism in total CFU (colony forming units), regardless of whether it is in the form of a culture, biomass or phytosanitary product formulation. The effective amount of the microorganism, biologically pure culture, biomass or phytosanitary product which can be applied on the plant for preventing, delaying or reducing infection caused by a nematode can vary within a broad range interval and be determined by the person skilled in the art taking into account the type of plant and the type of nematode, among other factors. In a particular embodiment, the plant is from the family *Solanaceae,* preferably a plant from the genus *Solanum,* even more preferably from the species *Solanum lycopersicum,* even more preferably from the "Marmande" variety, and the nematode is a nematode from the genus *Meloidogyne,* more preferably from the species *Meloidogyne javanica,* in which case the effective amount of the microorganism to be used is comprised between 1x10⁵ CFU and 1x10¹² CFU, preferably between 1x10⁷ CFU and 1x10¹¹ CFU.

The effective amount can be applied in a single administration or in several administrations. When it is applied in several administrations, said administrations can be 2, 3, 4, 5, 6, 7, 8, 9 or 10 applications in which application 4 is applied 10-20 days after application 3, and more preferably 15-20 days after application 3. In a particular embodiment, the effective amount of the microorganism, biologically pure culture, biomass or phytosanitary product is carried out in several applications: it is applied in a first application before transplanting the plant, in a second application after transplanting the plant, and at least a number "n" of applications, wherein "n" is an integer comprised between 1 and 10, wherein each of said "n" applications is applied between 10 and 20 days after the preceding application, preferably between 15-20 days after the preceding application.

In an even more particular embodiment, the effective amount of the microorganism, biologically pure culture, biomass or phytosanitary product is performed in three applications: a first application before transplanting the plant, a second application after transplanting, and the third application after the second application. In a more particular embodiment, the second application is performed between 10 and 20 days after transplanting, preferably between 5 and 16 days, more preferably 8 days after transplanting. In a more particular embodiment the third application is performed between 10 and 20 days after the second application, more preferably 15 days after the second application.

In an even more particular embodiment, the effective amount of the microorganism, biologically pure culture, biomass or phytosanitary product is performed in four applications: a first application before transplanting the plant, a second application after transplanting the plant, a third application after the second application and a fourth application after the third application. In a still more particular embodiment, the third application is performed between 10 and 20 days after the second application, preferably 15 days after the second application. In a still more particular embodiment, the fourth application is performed between 10 and 20 days after the third application, preferably 15 days after the third application.

The effective amount of the microorganism, biologically pure culture, biomass or phytosanitary product can be applied on the plant, on the seed of the plant, in the soil surrounding the plant or on a nematode susceptible of infecting the plant.

If it is applied on the plant, the application can be made on the entire plant or on some parts of the plant, for example, at the base of the stem, in the area occupied by the roots, tubercles and bulbs. The effective amount of the microorganism, biologically pure culture, biomass or phytosanitary product can be applied on the plant by any suitable technique or method, using suitable apparatuses, equipment and devices. The person skilled in the art knows the techniques and methods, as well as suitable apparatuses, equipment and devices for applying biological control agents on plants. In a particular embodiment, the microorganism, culture or biomass is formulated in the form of a phytosanitary product suitable for administration to a plant by any conventional technique or method and using any conventional device, for example, by spraying, dusting, or by any other suitable agricultural administration method, for example by means of baiting, incorporating into the soil or in automated or manual irrigation systems.

The application can also take place on the seed of the plant. As it is used herein, the term "seed" refers to any resting stage of a plant that is physically separated from the vegetative phase of the plant and/or that can be stored for more or less prolonged periods of time and/or that can be used to make another individual plant of the same species grow again. In this case, the application can be done before or while the seed is planted in the soil. The person skilled in the art knows suitable techniques for administering a biological control agent such as the microorganism, culture, biomass or phytosanitary product of the invention to a seed of a plant.

The application can also take place in the soil surrounding the plant. In a particular embodiment, administration is performed close to the neck of the root of the plant. The person skilled in the art knows suitable techniques for administering a biological control agent such as the microorganism, culture, biomass or phytosanitary product of the invention to the soil surrounding a plant.

The application can also take place on a nematode susceptible of infecting the plant. In that case, the application of the microorganism, culture, biomass or phytosanitary product on the nematode susceptible of infecting the plant is carried out by contacting said nematode with the microorganism, culture, biomass or phytosanitary product by means of any of the techniques or methods described in relation to the method for biologically controlling a nematode according to the seventh aspect.

### (ii) Planting a seed of said plant supplemented with a microorganism, biologically pure culture, biomass or phytosanitary product.

The seed that has been supplemented with a microorganism, biologically pure culture, biomass or phytosanitary product has previously been described.

As it is used herein, the term "planting" refers to placing a seed in prepared terrain so that it germinates, giving rise to the development of a plant. The supplemented seed of the invention can be planted by means of any suitable technique for planting seeds of a plant. Said techniques are known by the person skilled in the art.

All the embodiments defined for the previous aspects of the invention are applicable to the eighth aspect.

Particularly, in an embodiment the microorganism from the species *Ensifer adhaerens* is E. *adhaerens* strain B742, deposited in the Spanish Type Culture Collection (CECT) with accession number CECT 8809. In another embodiment the microorganism from the species *Ensifer adhaerens* is E. *adhaerens* strain B758, deposited in the Spanish Type Culture Collection (CECT) with accession number CECT 8810. In another embodiment the microorganism from the species *Ensifer adhaerens* is E. *adhaerens* strain B760, deposited in the Spanish Type Culture Collection (CECT) with accession number CECT 8808. In another embodiment the microorganism from the species *Ensifer adhaerens* is E. *adhaerens* strain TA12-B, deposited in the German Collection of Microorganisms and Cell Cultures (DSMZ) with accession number DSMZ 23677. In another embodiment the microorganism from the species *Ensifer adhaerens* is a combination of two or more of E. *adhaerens* strain B742, *E. adhaerens* strain B758, *E. adhaerens* strain B760 and E. *adhaerens* strain TA12-B.

In a ninth aspect, the invention relates to a method for treating a plant infected by a nematode comprising applying an effective amount of a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to the fifth aspect, on said plant or in the soil surrounding said plant.

As it is used herein, the expression "treating a plant infected by a nematode" refers to performing any activity aimed at holding up, stopping or reducing progression of the infection caused by a nematode, or improving at least one symptom of said infection. Holding up or stopping progression of the infection is understood to mean obtaining beneficial or desired results, including but not limited to reducing symptoms, reducing disease duration, stabilizing pathological states (specifically for preventing additional impairment), delaying progression of the disease, improving the pathological state and remission (both partial and complete). Control of progression of the disease also involves prolonging survival, compared with the survival that is expected if treatment is not applied.

The terms "plant", "nematode" and "effective amount" have previously been described. The methods for applying the microorganism, culture, biomass or phytosanitary product on the plant or in the soil surrounding the plant have previously been described.

The particular embodiments of the method of the eighth aspect also apply to the ninth aspect.

In a particular embodiment, the plant treated by means of the method of the ninth aspect belongs to the family *Solanaceae.*

In a particular embodiment of the method of the ninth aspect, the effective amount of a microorganism, a biologically pure culture, a biomass, or a phytosanitary product is applied a first time before transplanting the plant and a second time after transplanting the plant.

In a particular embodiment, the method according to the eighth or ninth aspect additionally comprises the use of a fungicide, nematicide or soil insecticide. The terms "fungicide", "nematicide" and "soil insecticide", as well as particular embodiments thereof, have previously been described.

### EXAMPLES

The following examples illustrate the invention and must be considered in an illustrative and non-limiting sense thereof.

### EXAMPLE 1

### Production and characterization of Ensifer adhaerens strains B742, B758 and B760

### Production

*Ensifer adhaerens* strains B742, B758 and B760 were isolated from a field managed according to ecological agriculture practices located in Barcelona, in February 2011.

Soil samples were homogenized using a 2mm pore size sieve. The samples were briefly air-dried. For bacteria isolation, 20 g of soil were suspended in 100 mL of phosphate buffer saline (KH₂PO₄ 2.72 g/L + K₂HPO₄ 3.48 g/L).The mixture was maintained in agitation at 300rpm for 30 minutes and later let to decant. Then serial dilutions from 10° to 10⁻⁵ were performed from supernatant and 100µL of every dilution were spread onto Potato Dextrose Agar culture medium (PDA: potato extract, 4 g/L; dextrose, 20 g/L; agar 15 g/L), nutritive agar (NA: meat extract, 1 g/L, yeast extract 2 g/L, soy peptone 5 g/L, sodium chloride 5 g/L and agar 15 g/L) and CZAPEK (Sucrose (30 g/L), NaNO₃ (3 g/L), MgSO₄.7H₂O (0.5 g/L), KCl (0.5 g/L), FeSO₄.7H₂O (0.01 g/L), K₂HPO₄ (1 g/L) and Agar (13.5 g/L)) plates. These plates were incubated in an incubator at 26°C and observed daily. Each of the colonies that grew (bacteria or fungi) was spread again in new plates with the same medium from which they came until obtaining pure cultures of the selected microorganisms. Finally, the purified isolates were separated from the medium by scraping the plate in soy peptone with 10% glycerol, giving rise to generation 0 of each microorganism, which was stored at -80°C.

### Characterization

The strains have been characterized at the morphological and molecular level. For morphological characterization, the bacteria were incubated in general medium [nutritive agar (NA)] at 2 temperatures (26°C and 37°C) and colony characteristics were observed.

### Morphological characterization:

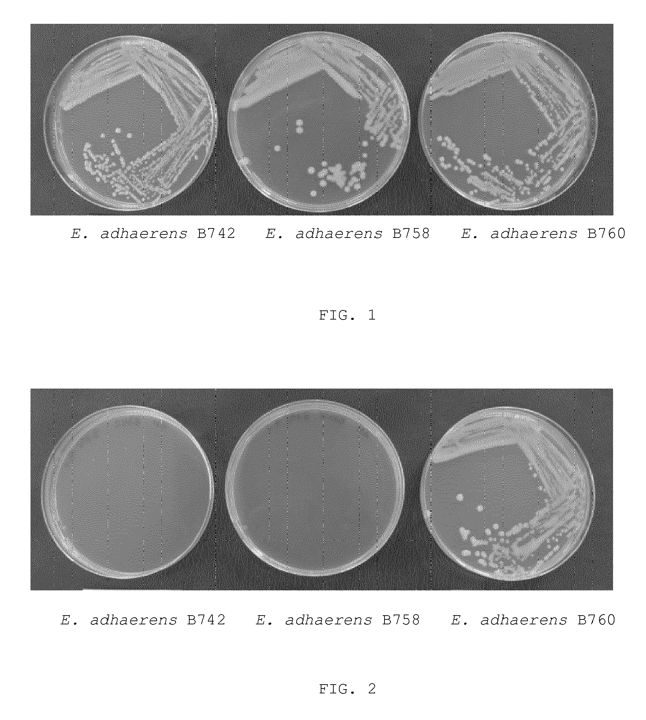

*Ensifer adhaerens* strains B758, B742 and B760 are gram-negative bacteria. They have colonies with a diameter of 10 mm after 96 hours of growth in Petri dishes with NA medium in a bacteriological incubator at a constant temperature of 26°C. The resulting colonies are white, medium-sized and circular colonies with a raised elevation and entire margin. When grown at 26°C, the colonies have an opaque appearance with a white color (Fig. *1**). Ensifer adhaerens* strain B760 is able to grow at 37°C but *Ensifer adhaerens* B742 and B758 strains not (Fig. 2). The morphology both at 26°C and 37°C is the same.

### Molecular characterization:

The molecular identification of *Ensifer adhaerens* strains B742, B758 and B760 was performed by means of amplifying 16S ribosomal DNA sub-unit with the use of the primers:
FORWARD: 8-f (AGTTTGATCCTGGCTCAG) (SEQ ID NO: 1) and
REVERSE: 1492-r (ACGGTTACCTTGTTACGACTT) (SEQ ID NO: 2).

The search for matching sequences was performed using BLAST (Basic Local Alignment Search Tool) software and the results showed that the amplified sequences were within the species *Ensifer adhaerens.*

The sequences obtained from the fragments amplified by these primers are SEQ ID NO: 3 for strain B742, SEQ ID NO: 4 for strain B758 and SEQ ID NO: 5 for strain B760. These sequences are shown below:

### B742 (SEQ ID NO: 3)

CGAGCGCCCCGCAAGGGGAGCGGCAGACGGGTGAGTAACGCGTGGGAATCTACCCTTTTCTACG
GAATAACGCAGGGAAACTTGTGCTAATACCGTATGAGCCCTTCGGGGGAAAGATTTATCGGAA
AGGATGAGCCCGCGTTGGATTAGCTAGTTGGTGGGGTAAAGGCCTACCAAGGCGACGATCCATA
GCTGGTCTGAGAGGATGATCAGCCACATTGGGACTGAGACACGGCCCAAACTCCTACGGGAGGC
AGCAGTGGGGAATATTGGACAATGGGCGCAAGCCTGATCCAGCCATGCCGCGTGAGTGATGAAG
GCCCTAGGGTTGTAAAGCTCTTTCACCGGTGAAGATAATGACGGTAACCGGAGAAGAAGCCCCG
GCTAACTTCGTGCCAGCAGCCGCGGTAATACGAAGGGGGCTAGCGTTGTTCGGAATTACTGGGC
GTAAAGCGCACGTAGGCGGACATTTAAGTCAGGGGTGAAATCCCGGGGCTCAACCCCGGAACTG
CCTTTGATACTGGGTGTCTAGAGTATGGAAGAGGTGAGTGGAATTCCGAGTGTAGAGGTGAAAT
TCGTAGATATTCGGAGGAACACCAGTGGCGAAGGCGGCTCACTGGTCCATTACTGACGCTGAGG
TGCGAAAGCGTGGGGAGCAAACAGGATTAGATACCCTGGTAGTCCACGCCGTAAACGATGAATG
TTAGCCGTCGGGCAGTTTACTGTTCGGTGGCGCAGCTAACGCATTAAACATTCCGCCTGGGGAG
TACGGTCGCAAGATTAAAACTCAAAGGAATTGACGGGGGCCCGCACAAGCGGTGGAGCATGTGG
TTTAATTCGAAGCAACGCGCAGAACCTTACCAGCCCTTGACATCCCGATCGCGGATTACGGAGA
CGTTTTCCTTCAGTTCGGCTGGATCGGAGACAGGTGCTGCATGGCTGTCGTCAGCTCGTGTCGT
GAGATGTTGGGTTAAGTCCCGCAACGAGCGCAACCCTCGCCCTTAGTTGCCAGCATTTAGTTGG
GCACTCTAAGGGGACTGCCGGTGATAAGCC

### B758 (SEQ ID NO: 4)

TGCAGTCGAGCGCCCCGCAAGGGGAGCGGCAGACGGGTGAGTAACGCGTGGGAATCTACCCTTT
TCTACGGAATAACGCAGGGAAACTTGTGCTAATACCGTATAAGCCCTTCGGGGGAAAGATTTAT
CGGGAAAGGATGAGCCCGCGTTGGATTAGCTAGTTGGTGGGGTAAAGGCCTACCAAGGCGACGA
TCCATAGCTGGTCTGAGAGGATGATCAGCCACATTGGGACTGAGACACGGCCCAAACTCCTACG

GGAGGCAGCAGTGGGGAATATTGGACAATGGGCGCAAGCCTGATCCAGCCATGCCGCGTGAGTG
ATGAAGGCCCTAGGGTTGTAAAGCTCTTTCACCGGTGAAGATAATGACGGTAACCGGAGAAGAA
GCCCCGGCTAACTTCGTGCCAGCAGCCGCGGTAATACGAAGGGGGCTAGCGTTGTTCGGAATTA
CTGGGCGTAAAGCGCACGTAGGCGGACATTTAAGTCAGGGGTGAAATCCCAGAGCTCAACTCTG
GAACTGCCTTTGATACTGGGTGTCTAGAGTATGGAAGAGGTGAGTGGAATTCCGAGTGTAGAGG
TGAAATTCGTAGATATTCGGAGGAACACCAGTGGCGAAGGCGGCTCACTGGTCCATTACTGACG
CTGAGGTGCGAAAGCGTGGGGAGCAAACAGGATTAGATACCCTGGTAGTCCACGCCGTAAACGA
TGAATGTTAGCCGTCGGGCAGTTTACTGTTCGGTGGCGCAGCTAACGCATTAAACATTCCGCCT
GGgGAGTACGGTCGCAAGATTAAAACTCAAAGGAATTGACGGGGGCCCGCACAAGCGGTGGAGC
ATGTGGTTTAATTCGAAGCAACGCGCAgAACCtTACCAGCCCTTGACATCCCGATCGCGGATTA
CGGAGACGTTTTCCTTCAgTTCGGCTGGATCGGAgACAGGTGCTGCATGGCTGTCGTCAGCTCG
TGTCGtGAGATGTTGGGTTAAGTCCC

### B760 (SEQ ID NO: 5)

AGTCGAGCGCCCCGCAAGGGGAGCGGCAGACGGGTGAGTAACGCGTGGGAATCTACCTTTTCT
ACGGAATAACGCAGGGAAACTTGTGCTAATACCGTATACGCCCTTCGGGGGAAAGATTTATCGG
GAAAGGATGAGCCCGCGTTGGATTAGCTAGTTGGTGGGGTAAAGGCCTACCAAGGCGACGATCC
ATAGCTGGTCTGAGAGGATGATCAGCCACATTGGGACTGAGACACGGCCCAAACTCCTACGGGA
GGCAGCAGTGGGGAATATTGGACAATGGGCGCAAGCCTGATCCAGCCATGCCGCGTGAGTGATG
AAGGCCCTAGGGTTGTAAAGCTCTTTCACCGGTGAAGATAATGACGGTAACCGGAGAAGAAGCC
CCGGCTAACTTCGTGCCAGCAGCCGCGGTAATACGAAGGGGGCTAGCGTTGTTCGGAATTACTG
GGCGTAAAGCGCACGTAGGCGGACATTTAAGTCAGGGGTGAAATCCCGGGGCTCAACCCCGGAA
CTGCCTTTGATACTGGGTGTCTAGAGTATGGAAGAGGTGAGTGGAATTCCGAGTGTAGAGGTGA
AATTCGTAGATATTCGGAGGAACACCAGTGGCGAAGGCGGCTCACTGGTCCATTACTGACGCTG
AGGTGCGAAAGCGTGGGGAGCAAACAGGATTAGATACCCTGGTAGTCCACGCCGTAAACGATGA
ATGTTAGCCGTCGGGCAGTTTACTGTTCGGTGGCGCAGCTAACGCATTAAACATTCCGCCTGGG
GAGTACGGTCGCAAGATTAAAACTCAAAGGAATTGACGGGGGCCCGCACAAGCGGTGGAGCATG
TGGTTTAATTCGAAGCAACGCGCAGAACCTTACCAGCCCTTGACATCCCGATCGCGGATTACGG
AGACGTTTTCCTTCAGTTCGGCTGGATCGGAGACAGGTGCTGCATGGCTGTCGTCAGCTCGTGT
CGTGAGATGTTGGGTTAAGTCCCGCAACGAGCGCAACCCTCGCCCTTAGTTGCCAGCATTTAGT
TGGGCACTCTAAGGGGACTGCCGG

To demonstrate that B742, B758 and B760 are different strains, a protocol to differentiation at strain level was carried out. Genomic DNA was extracted with Zymo Kit according to manufacturer's instructions. DNA fingerprinting methods used included REP and ERIC PCR (Versalovic, J., Schneider, M., De Bruijn, F. J., & Lupski, J. R. (1994). Genomic fingerprinting of bacteria using repetitive sequence-based polymerase chain reaction. Methods in molecular and cellular biology, 5(1), 25-40; Ateba, C. N., & Mbewe, M. (2013). Determination of the genetic similarities of fingerprints from Escherichia coli O157: H7 isolated from different sources in the North West Province, South Africa using ISR, BOXAIR and REP-PCR analysis. Microbiological research, 168(7), 438-446.). Primers used for typing E. *adhaerens* were:
REP 1R 5'-IIIICGICGICATCIGGC-3' (SEQ ID NO: 6)
Rep2I 5'-ICGICTTATCIGGCCTAC-3' (SEQ ID NO: 7) and
ERIC 1 5'-AGTAAGTGACTGGGGTGAGC-3' (SEQ ID NO: 8)
ERIC 2 5'- ATGTAAGCTCCTGGGGATTCAC-3' (SEQ ID NO: 9)

DNA amplification was conducted on an Eppendorf thermocycler programmed for an initial denaturation step at 95°C (7 minutes), followed by 30 cycles of denaturation step for 6 minutes at 90°C, annealing for 1 minute at the appropriated temperature (REP at 40°C and ERIC at 52°C), extension for 8 minutes at 65°C, and a final extension for 8 minutes at 65°C. Samples were maintained at 4°C until electrophoretic separation of amplification products. The PCR products were electrophoresed in 1.5% agarose gel in Tris-borate buffer. The gels were stained with GEL RED® and visualized on a UV light transilluminator.

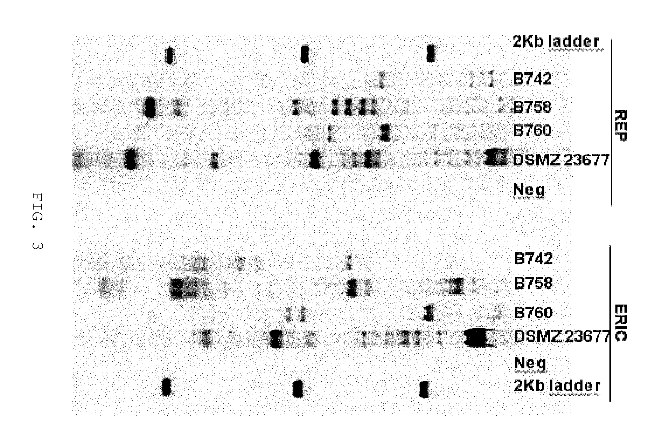

The 4 isolates typed using the REP PCR analysis produced 4 different patterns with bands of different size (Fig. 3). The results of this study allowed concluding that *Ensifer adhaerens* B742, B758, B760 and DSMZ 23677 are different isolates.

### EXAMPLE 2

### In vitro nematicidal activity of Ensifer adhaerens strain B758 against Meloidogyne javanica eggs

### Materials and Methods

The nematicidal activity of *Ensifer adhaerens* against *Meloidogyne javanica* eggs was evaluated by *in vitro* assays. Nematode eggs were obtained from *M. javanica* infected tomato roots. Aliquots (200-300 µL) of the egg suspensions containing 350 eggs were pipetted onto the hatching chambers, which had previously been placed in a sterile micro-well plate (with one hatching chamber per well) containing 3 mL of sterile distilled water. The plate was then left for 24 h to collect any juvenile (J₂) present in the egg suspension. The chambers were then transferred to a new micro-well plate and the biological control agent *Ensifer adhaerens* strain B758 was added at 1.19x10¹⁰ CFU's/mL. The wells containing sterile distilled water served as controls and a chemical control (fenamiphos) was included too. Eight replications were prepared per treatment. The plates were incubated at 26°C in darkness, and the number of hatched J₂ was recorded after 1, 7, 15 and 21 days. At each time, the hatching chambers were removed from the wells and placed in new ones containing fresh sterile distilled water.

### Results and discussion

**Table 1.- Efficacy (%) corrected with respect to the control of the chemical used as a reference and E. adhaerens strain B758 against M. javanica eggs.**

| **Treatments** | **Efficacy (%)** |
|---|---|
| Fenamiphos | 90.89 |
| *E. adhaerens* B758 | 79.12 |

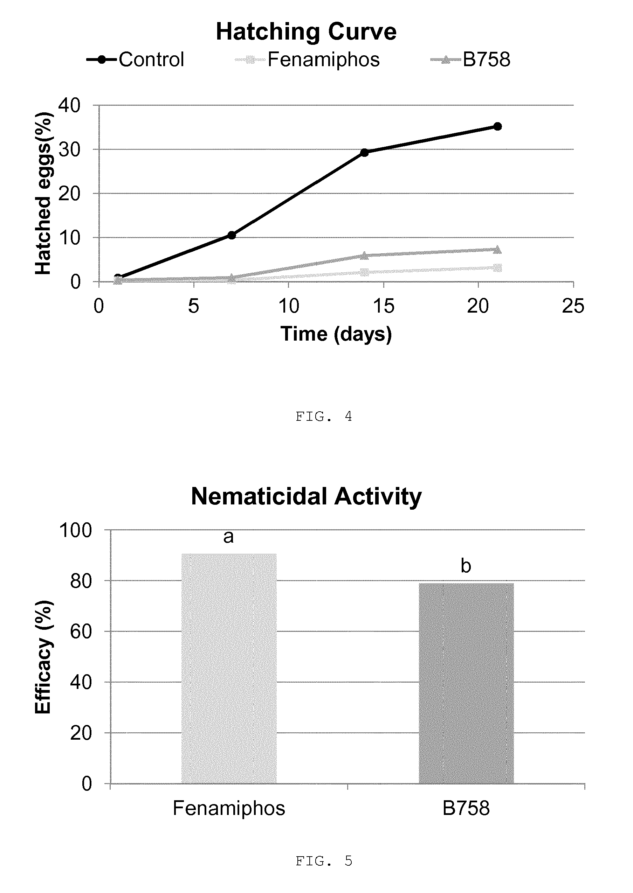

In the follow-up for the hatching of *M. javanica* eggs for the 21 days the assay lasted, treatment with E. *adhaerens* strain B758 showed a significant reduction with respect to the control (Fig. 4).

Three weeks after applying the different treatments, 35.27% of the untreated eggs (control) hatched, whereas 7.36%of those treated with *E*. *adhaerens* strain B758 hatched and 3.21% of those treated with fenamiphos hatched.

Therefore, *E. adhaerens* strain B758 has 79.12% efficacy in reducing hatching with respect to the control under *in vitro* conditions, whereas in the reference chemical, efficacy in reducing hatching was 90.89% (Fig. 5).

### EXAMPLE 3

### In vivo nematicidal activity of Ensifer adhaerens strain B758 against Meloidogyne javanica

### Materials and Methods

Seeds of commercial tomato cultivar "Marmande" were sown in a 36-seed-bed (5x5x6cm) filled with sterile peat (Compo®) and kept under controlled conditions (22°C; 60% RH; 14:10h light:dark) in a Phytotron (Fitoclima 1200, ARALAB) for 4 weeks. Tomato seedlings cv. Marmande were inoculated with 10 mL/plant of a suspension containing 2.24x10⁸ CFUs/mL of the Biological Control Agent (BCA) *Ensifer adhaerens* strain B758.

Seventy two hours (72 h) after the first application (A) of the BCA, the seedlings were transplanted singly to 1.5 L pots containing a mixture of stem-sterilized river sand, peat and perlite (4:2:1 v/v) (autoclaved twice in two separated days, 121 °C, 1 h).

Twenty four hours (24 h) after transplanting the seedlings to the pots, 1500 *Meloidogyne javanica* juveniles (J₂) which were routinely maintained on tomato cv. Marmande plants, were inoculated to each plant by pipetting 4 mL of a juvenile suspension into 4 holes made around the base of the plant.

One week after inoculation with nematodes, a second application (B) of 2.6x10⁸ CFUs/mL of the BCA was made by pipetting 15 mL of -bacterial suspension to the base of the plant.

Finally, 15 days after second application, a third application (C) of *Ensifer adhaerens* strain B758 was performed at a concentration of 3.92x10⁷ CFUs/mL by the same method as described above.

An absolute control with only nematode inoculum and a chemical standard (Nemacur 40 LE, active ingredient: Fenamiphos 40% emulsifiable concentrate p/v) was included as a reference treatment and applied at commercial dose (12 L/ha) and at the same application timing and volume than the biocontrol agent treatments (A-B-C). Each treatment consisted of 10 replications.

All the plants were kept in a walking-in growth chamber (Fitoclima 15000 EH, ARALAB) at 18-22 °C; 60-70% RH and 15h:9h (day:night photoperiod) for eight weeks from the transplant. After that, tomato plants were harvested and growth parameters along with nematode reproduction and fertility were determined.

### Results and discussion

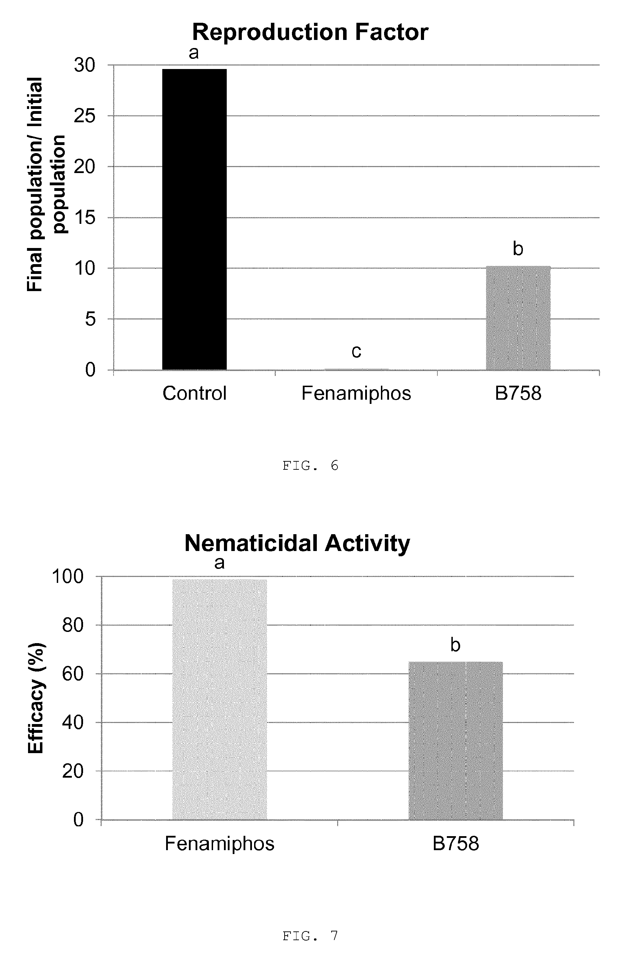

Reproduction factor was determined (Fig. 6). The results shown significant differences between control and treatments with E. *adhaerens* B758 and the chemical product fenamiphos used as a reference.

**Table 2.- Efficacy (%) corrected with respect to the control of the chemical used as a reference and E. adhaerens strain B758 against M. javanica.**

| **Treatments** | **Efficacy (%)** |
|---|---|
| Fenamiphos | 99.28 |
| *E. adhaerens* B758 | 65.38 |

Therefore, *E*. *adhaerens* strain B758 has 65.38% nematicidal activity with respect to the control under *in vivo* conditions, whereas in the reference chemical efficacy was 99.28% (Fig. 7).

### EXAMPLE 4

### Study of nematicidal activity of different strains of Ensifer adhaerens against Meloidogyne javanica eggs

### Materials and Methods

The nematicidal activity of *Ensifer adhaerens* against *Meloidogyne javanica* eggs was evaluated by *in vitro* assays. The strains of *Ensifer adhaerens* assayed were B742, B758, B760 and DSMZ 23677. *Ensifer adhaerens* strains B742, B758 and B760 belong to Futureco Bioscience Microorganism Collection and were previously isolated as described in Example 1, and DSMZ 23677 was acquired to German Collection of Microorganisms and Cell Culture.

Nematode eggs were obtained from *M. javanica* infected tomato roots. Aliquots (200-300 µL) of the egg suspensions containing 370 eggs were pipetted onto the hatching chambers, in which 2 g of previously sterilized soil had been added. Every hatching chamber had previously been placed in a sterile micro-well plate (Nunclon™Surface multiwell plates, Nunc, Denmark) (with one hatching chamber per well) containing 3 mL of sterile distilled water. The plate was then left for 24 h to collect any juvenile (J₂) present in the egg suspension. The chambers were then transferred to a new micro-well plate and the different strains of *Ensifer adhaerens* (B742, B758, B760 and DSMZ 23677) were added to the concentration detailed in Table 3.

**Table 3.- Concentration of strains of E. adhaerens assayed.**

| ***Ensifer adhaerens* strain** | **Concentration CFUs/mL** |
|---|---|
| B760 | 9.00E+08 |
| DSMZ 23677 | 5.30E+08 |
| B758 | 8.90E+08 |
| B742 | 8.30E+08 |

Wells containing sterile distilled water as controls were included and wells containing a chemical control (fenamiphos) were included too. Eight replications were prepared per treatment. The plates were incubated at 26°C in darkness for 3 weeks and the number of hatched J₂ was recorded 7, 15 and 21 days after applying *Ensifer adhaerens* strains. At each time, the hatching chambers were removed from the wells and placed in new ones containing fresh sterile distilled water.

### Results and discussion

**Table 4.- Efficacy (%) corrected with respect to the control of the chemical used as a reference (fenamiphos) and of different E. adhaerens strains against M. javanica eggs.**

| **Treatments** | **Efficacy** |
|---|---|
| Fenamiphos | 99.57 |
| *E. adhaerens* B742 | 48.19 |
| *E. adhaerens* B758 | 50.53 |
| *E. adhaerens* B760 | 42.07 |
| *E. adhaerens* DSMZ 23677 | 12.06 |

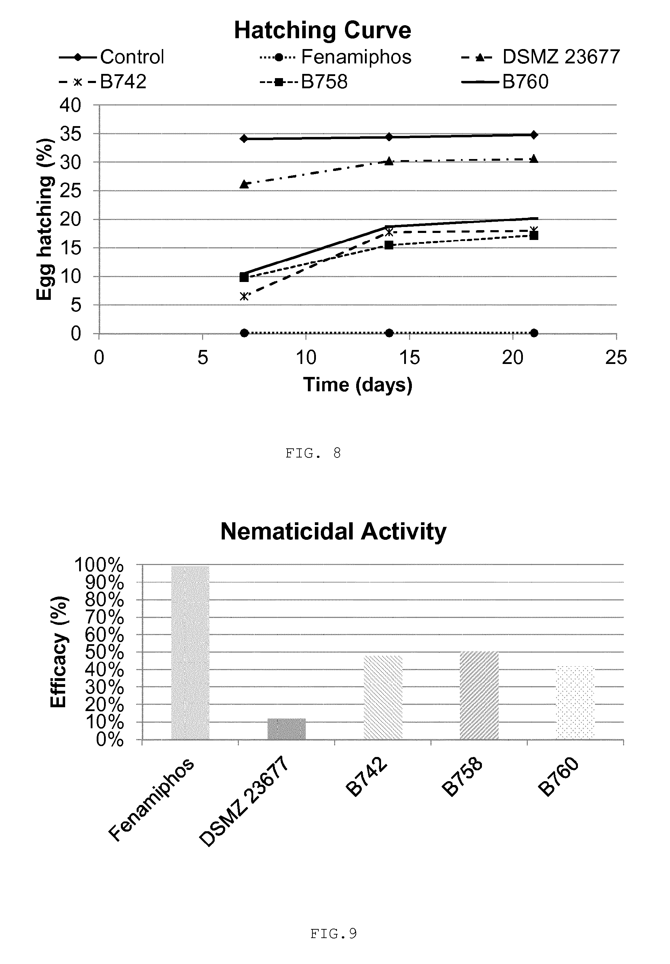

In the follow-up for the hatching of *M. javanica* eggs for the 21 days the assay lasted, treatment with all the E. *adhaerens* strains showed nematicidal activity (Fig. 8).

Three weeks after applying the different treatments,34.8% of the untreated eggs (control) hatched, whereas 18.04% of those treated with *E*. *adhaerens* strain B742 hatched, 17.23% of those treated with *E*. *adhaerens* strain B758 hatched, 20.17% of those treated with *E*. *adhaerens* strain B760 hatched and 30.62% of those treated with *E*. *adhaerens* DSMZ 23677 strain hatched.

Therefore, the efficacy in reducing hatching with respect to the control under *in vitro* conditions is 48.19% for E. *adhaerens* strain B742, 50.53% for E. *adhaerens* strain B758, 42.07% for E. *adhaerens* strain B760, and 12.06% for E. *adhaerens* DSMZ 23677 strain (Fig. 9).

### CONCLUSIONS

The results obtained show that *Ensifer adhaerens* strain B758 presents significant efficacy against eggs and juveniles of *M. javanica* both in *in vitro* and *in vivo* tests.

*Ensifer adhaerens* strain B758 presents an efficacy of 79.12% in *in vitro* test and an efficacy of 65.38% in *in vivo* test with high pathogen pressures.

All strains of *Ensifer adhaerens* evaluated in *in vitro* assays have demonstrated nematicidal activity against eggs and juveniles of *M. javanica.* The strains B742, B758 and B760 present more activity than strain DSMZ 23677.

The use of *Ensifer adhaerens* as bionematicide for the control of phytopathogenic nematodes or into an integrated pest management program, decreases residues and reduce developing nematode resistance to this control product.

### DEPOSIT OF BIOLOGICAL MATERIAL

*Ensifer adhaerens* strain B742 has been deposited in the Spanish Type Culture Collection (CECT) (Edificio 3 CUE, Parc Cientific Universitat de Valencia, Catedrático Agustin Escardino, 9; 46980 Paterna, Valencia, Spain) under the conditions set forth in the Budapest Treaty. The deposit took place on January 13, 2015 and the number assigned to said deposit was CECT 8809.

*Ensifer adhaerens* strain B758 has been deposited in the Spanish Type Culture Collection (CECT) (Edificio 3 CUE, Parc Cientific Universitat de Valencia, Catedrático Agustin Escardino, 9; 46980 Paterna, Valencia, Spain) under the conditions set forth in the Budapest Treaty. The deposit took place on January 13, 2015 and the number assigned to said deposit was CECT 8810.

*Ensifer adhaerens* strain B760 has been deposited in the Spanish Type Culture Collection (CECT) (Edificio 3 CUE, Parc Cientific Universitat de Valencia, Catedrático Agustin Escardino, 9; 46980 Paterna, Valencia, Spain) under the conditions set forth in the Budapest Treaty. The deposit took place on January 13, 2015 and the number assigned to said deposit was CECT 8808.

## Claims

1. A microorganism from the species *Ensifer adhaerens* having nematicidal activity or a mutant of said microorganism maintaining said nematicidal activity selected from the group consisting of:
(i) *E. adhaerens* strain B742, deposited in the Spanish Type Culture Collection (CECT) with accession number CECT 8809,
(ii) *E. adhaerens* strain B758, deposited in the Spanish Type Culture Collection (CECT) with accession number CECT 8810, and
(iii) *E. adhaerens* strain B760, deposited in the Spanish Type Culture Collection (CECT) with accession number CECT 8808.

2. The microorganism according to claim 1, wherein said microorganism has nematicidal activity on a nematode from the species *Meloidogyne javanica.*

3. A biologically pure culture of a microorganism according to any of claims 1 or 2.

4. A method for obtaining a biomass of the microorganism according to any of claims 1 or 2 comprising culturing said microorganism under conditions suitable for growth.

5. A biomass of the microorganism according to any of claims 1 or 2, obtainable by means of the method of claim 4.

6. A phytosanitary product comprising a microorganism from the species *Ensifer adhaerens* having nematicidal activity or a mutant of said microorganism maintaining said nematicidal activity, or a biologically pure culture of said microorganism or mutant thereof, or a biomass of said microorganism or mutant thereof, and an agriculturally acceptable excipient.

7. A supplemented seed comprising a seed, and furthermore a microorganism from the species *Ensifer adhaerens* having nematicidal activity or a mutant of said microorganism maintaining said nematicidal activity, or a biologically pure culture of said microorganism or mutant thereof, or a biomass of said microorganism or mutant thereof, or a phytosanitary product according to claim 6.

8. The supplemented seed according to claim 7, wherein the seed is one of a plant from the family *Solanaceae.*

9. A method for biologically controlling a nematode comprising applying to said nematode a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to claim 6.

10. A method for preventing plant infection caused by a nematode comprising applying an effective amount of a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to claim 6, on said plant, on the seed of said plant, in the soil surrounding said plant or on a nematode susceptible of infecting said plant, or alternatively planting a seed of said plant supplemented with a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to claim 6.

11. A method for treating a plant infected by a nematode comprising applying an effective amount of a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to claim 6, on said plant or in the soil surrounding said plant.

12. The method according to any of claims 9 to 11, wherein the microorganism from the species *Ensifer adhaerens* is selected from the group consisting of:
(i) *E. adhaerens* strain B742, deposited in the Spanish Type Culture Collection (CECT) with accession number CECT 8809,
(ii) *E. adhaerens* strain B758, deposited in the Spanish Type Culture Collection (CECT) with accession number CECT 8810,
(iii) *E. adhaerens* strain B760, deposited in the Spanish Type Culture Collection (CECT) with accession number CECT 8808,
(iv) *E. adhaerens* strain TA12-B, deposited in the German Collection of Microorganisms and Cell Cultures (DSMZ) with accession number DSMZ 23677, and
(v) a combination of two or more of (i), (ii), (iii) and (iv).

13. The method according to any of claims 9 to 12, wherein the microorganism has nematicidal activity on a nematode from the species *Meloidogyne javanica.*

14. The method according to any of claims 10 to 13, wherein the plant belongs to the family *Solanaceae.*

15. The method according to any of claims 10 to 14, wherein the effective amount of a microorganism from the species *Ensifer adhaerens* having nematicidal activity or a mutant of said microorganism maintaining said nematicidal activity, a biologically pure culture of said microorganism or mutant thereof, a biomass of said microorganism or mutant thereof, or a phytosanitary product according to claim 6, is applied in a first application before transplanting the plant, in a second application after transplanting the plant, and at least a number "n" of applications, wherein "n" is an integer comprised between 1 and 10, wherein each of said "n" applications is applied between 10 and 20 days after the preceding application.
